Europäisches Patentamt

European Patent Office

Office européen des brevets

(19)

(11) Publication number : **0 604 224 A1**

# (12) EUROPEAN PATENT APPLICATION

(21) Application number : 93310446.5

(22) Date of filing : 22.12.93

(51) Int. Cl.⁵ : **C12N 15/12,** C07K 13/00,
G01N 33/68, A01K 67/027

(30) Priority : 22.12.92 US 995627
28.05.93 US 70163

(43) Date of publication of application :
29.06.94 Bulletin 94/26

(84) Designated Contracting States :
AT BE CH DE DK ES FR GB GR IE IT LI LU NL
PT SE

(71) Applicant : **ELI LILLY AND COMPANY**
Lilly Corporate Center
Indianapolis Indiana 46285 (US)

(72) Inventor : **Hsiung, Hansen Maxwell**
14436 Jeremy Drive
Carmel, Indiana 46032 (US)
Inventor : **Smith, Dennis Paul**
4622 Eden Court
Indianapolis, Indiana 46254 (US)
Inventor : **Zhang, Xing-Yue**
6450 Camden Street
Indianapolis, Indiana 46227 (US)

(74) Representative : **Hudson, Christopher Mark et al**
Erl Wood Manor
Windlesham Surrey GU20 6PH (GB)

(54) Porcine GRF receptor.

(57)   The instant invention provides the porcine GRF receptor protein. The invention also provides DNA compounds encoding the pGRF receptor, recombinant DNA cloning and expression vectors comprising said DNA sequence, and recombinant host cells comprising said vectors. The invention also provides methods of making the pGRF receptor in recombinant and solid phase systems. The invention also provides methods of using the pGRF receptor in assay systems. The invention also provides methods of using said DNA sequences as probes to isolate and characterize GRF receptors and corresponding DNA sequences from other species.

EP 0 604 224 A1

Peptide hormones act as messengers on target tissues stimulating the target organ to respond to a physiological conditions sensed, directly or indirectly, from the brain. A variety of diseases are known to result from abnormalities in the quantity, stability, or activity of endogenously produced peptide hormones. Some types of these diseases are amenable to treatment by hormone supplementation. Artificially increasing circulating levels of growth hormone (GH) is well known to be effective not only in treating GH deficiency disorders but also as a performance enhancing supplement in mammals. Peptide hormones, classically isolated from natural sources and more recently produced using recombinant DNA technology, are commercially available for the treatment of a variety of diseases.

Artificially increasing circulating levels of growth hormone (GH) is well known to be effective not only in treating GH deficiency disorders but also as a performance enhancing supplement in mammals. The mechanism of action of peptide hormones and their corresponding therapeutic potential cannot be divorced from the complex cascade which mediates their action. Maintenance of the balance between synthesis and catabolism, activation and repression or energy usage and storage allow the organism to adapt to environmental stress but return to a basal state once the stimulus is removed.

The desired result of increasing GH levels in circulation may be achieved by modifying any of the levels of control of growth hormone. The peptide hormone, Growth Hormone Releasing Factory (GRF), also commonly known in the art as somatocrinin, Growth Hormone Releasing Hormone (GHRH), and Growth Releasing Hormone (GRH) has been demonstrated to increase the circulatory levels of growth hormone in vivo (Rivier, et al. (1982) Nature 300:276). The release of GH from the pituitary glands of mammals is effected by the combined action of GRF and somatostatin. (Devesa, et al. (1992) TEM 3:175-181.) GRF is released from the hypothalamus into the bloodstream where it migrates to the pituitary gland. It is believed that GH is released from the pituitary as GRF binds to a receptor on the cell surface, termed a GRF receptor. The binding of GRF to the GRF receptor activates the signal transduction cascade which eventually results in GH release.

The instant invention provides a protein, the porcine growth hormone releasing factor(pGRF) receptor whose activity is intimately tied to the cascade of events which stimulate growth hormone effects in an organism. A number of G-protein coupled receptors have been cloned, for example, the receptors for mouse gonadotropin release hormone(Tsutsumi, et al., 1991, Molecular Endocrinology 6:1163-1169), adenylate cyclase activating polypeptide (Arimura, A., 1992, TEM 3:288-294, lutropin-choriogonadotropin (McFarland, et al., 1989, Science 245: 494-499), thyrotropin releasing hormone (Zhao, et al., 1992, Endocrinology 130:3529-3536), human and rat dopamine (Zhou, et al., 1990, Nature 347:76-80), glucagon-incretin hormone glucagon-like peptide 1(Thorens 1992, PNAS 89:8641-8645), calcitonin (Lin, et al., 1991, Science 254:1022-1024) parathyroid hormone and parathyroid hormone-related peptide (Abou-Samra, et al., 1992, PNAS 89:2732-2736), endothelin 1 (Lin, et al., 1991 PNAS 88:3185-3189), secretin (Ishihara, et al., 1991, EMBO J. 10:1635-1641), and vasoactive intestinal peptide (Ishihara et al., 1992, Neuron 8:811-819).

Although the art has attempted to purify GRF receptors, these preparations have been insufficiently pure to permit determination of the sequence of the GRF receptor. Surprisingly, the present inventors have overcome the inability of the prior art to provide the GRF receptor in substantially pure form. The present invention also provides methods to purify the GRF receptor and DNA sequences encoding same, and to produce useful quantities of each using recombinant DNA techniques.

Specifically, the instant invention in provides in its various embodiements: the pGRF receptor and functional analogs thereof; fusion proteins comprising the pGRF receptor or fragments thereof with or without a signal peptide sequence; DNA sequences encoding the pGRF receptor; a method for the recombinant production of the pGRF receptor; recombinant DNA vectors comprising a DNA sequence encoding the pGRF receptor; transformed host cells useful in the recombinant expression of the pGRF receptor; a screening system comprising the pGRF receptor useful for determining agents which stimulate or inhibit the action of the pGRF receptor; a bioactivity assay system comprising eucaryotic host cells expressing the pGRF receptor useful for quantifying the level of stimulation or repression of the pGRF activity in reponse to test compounds; antibodies against the pGRF receptor useful in imaging or diagnostic applications; and transgenic animals which express the pGRF receptor.

The various embodiements of the present invention have been accomplished by the identification and cloning the cDNA sequence encoding the pGRF receptor; incorporating that DNA sequence into a recombinant DNA vector; transforming a suitable host with the vector including that DNA sequence; expressing the pGRF receptor in such a host; and recovering the pGRF receptor so produced. Similarly, the present invention makes it possible to produce the pGRF receptor and analogs thereof by recombinant techniques, as well as providing products and methods related to the GRF receptor.

A series of drawings accompany the present disclosure to ensure complete understanding of the invention. The restriction site and function maps presented in the accompanying drawings are approximate representations of the recombinant DNA vectors discussed herein. The restriction site information is not exhaustive;

therefore there may be more restriction sites of a given type on the vector than are illustrated in the drawings.

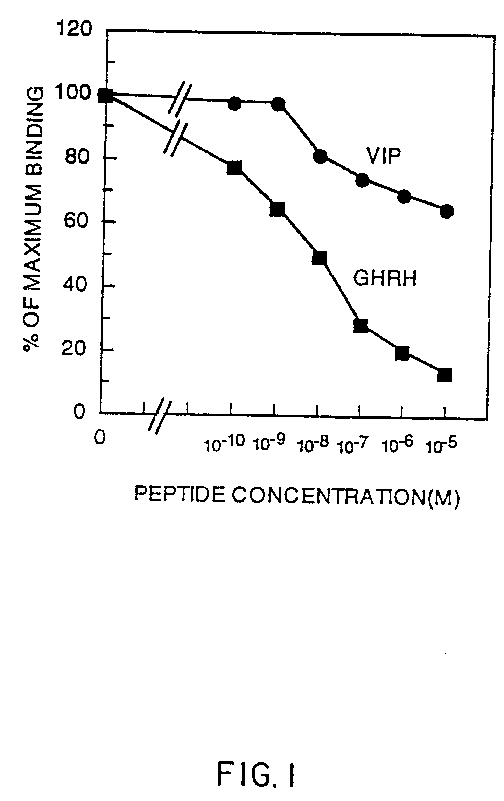

Figure 1 -- A radioreceptor assay that shows that the binding specificity of the pGRF receptor. Unlabelled secretin, pGRF, and vasoactive intestinal peptide (VIP) was used to compete with $^{125}$I-GRF binding to pGRF receptor expressing cells, 293/GS-12.

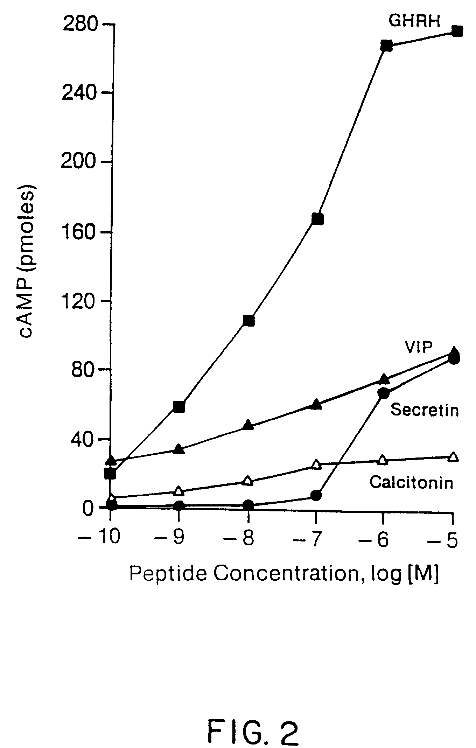

Figure 2 -- A graphical representation of the the levels of cAMP produced in 293 cells which express the GRF receptor, 293/G5-12, in response to calcitonin, secretin, VIP, and pGRF(1-44)OH.

Figure 3 -- A restriction site and function map of plasmid pRc/CMV.

Figure 4 -- A hydrophobicity plot of the GRF receptor.

A variety of research materials were used in the course of research leading to the instant inventions and for convenience the sources of those materials will bbe provided in the section which follows.

Restriction endonucleases and other enzymes (ligase, kinase, phosphatase, polymerases) are commercially available from Boehringer-Mannheim (9115 Hague Road, Indianapolis, IN). Peptide hormones human calcitonin, porcine secretin, porcine vasoactive intestinal polypeptide (VIP), PACAP, GIP, PTH, PHI, PHM are commercially available from Peninsula Laboratories (611 Taylor Way, Belmont, California 94002-4041). Porcine GRF (1-44)-OH, Glucagon and Glucagon-like peptide 1 were synthesized by conventional techniques from their known published amino acid sequences. [125I]human GRF (1-44)-amide is commercially available from Amersham Corporation, 2636 South Clearbrook Drive, Arlington Heights, IL 60005.as catalog #IM.180). Antibiotic Geneticin G-418 is commercially available from Gibco-BRL . Liquid nitrogen-frozen porcine tissues used for RNA isolation were obtained from Pel-Freez, 205 N. Arkansas, P.O. Box 68, Rogers Arkansas 72757.

For purposes of the present invention as disclosed and claimed herein, the following terms are defined as follows:

Analog -- a compound which is structurally similar to another. When used in reference to polypeptides it refers to primary, secondary, or tertiary structure.

Base pair (bp) -- refers to DNA or RNA. The abbreviations A,C,G, and T correspond to the 5'-monophosphate forms of the nucleosides (Deoxy)adenosine, (deoxy)cytidine, (deoxy)guanosine, and (deoxy)thymidine, respectively, when they occur in DNA molecules. The abbreviations U,C,G, and T correspond to the 5'-monophosphate forms of the nucleosides uridine, cytidine, guanosine, and thymidine, respectively when they occur in RNA molecules. In double stranded DNA, base pair may refer to a partnership of A with T or C with G. In a DNA/RNA, heteroduplex base pair may refer to a partnership of T with U or C with G.

Control region -- refers to specific sequences at the 5' and 3' ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence which may be the signal for addition of the poly A$^+$ tail to the 3' end of the transcribed mRNA.

Dephosphorylation -- refers to the removal of the N-terminal 5' phosphates by treatment with bacterial alkaline phosphatase (BAP) or calf intestinal alkaline phosphatase (CIAP). This procedure prevents the two restriction cleaved ends of a DNA fragment from "circularizing" or forming a closed loop that would impede insertion of another DNA fragment at the restriction site. Procedures and reagents for dephosphorylation are conventional. Maniatis, T. et al., Molecular Cloning pp. 133-134 (1982).

Digestion -- of DNA refers to catalytic cleavage of the DNA with a restriction enzyme that acts only at certain sequences in the DNA. The various restriction enzymes used herein are commercially available and their reaction conditions, cofactors, and other requirements were used as would be known to one of ordinary skill in the art. Appropriate buffers and substrate amounts for particular restriction enzymes are specified by the manufacturer.

Filling or blunting -- the procedures by which the single stranded end in the cohesive terminus of a restriction enzyme-cleaved nucleic acid is converted to a double strand. This procedure eliminates the cohesive ("sticky") end and forms a blunt end. This process is used to convert a restriction-cut end that may be cohesive with a terminus compatible with any blunt-cutting restriction endonuclease or other filled cohesive terminus. Typically, blunting or filling is achieved by the use of the Klenow fragment of DNA polymerase I by procedures well known in the art.

Functional analog -- refers to a molecule having similar functional properties but a modified structure relative to the naturally occurring form of that molecule or compound. Functional analogs include fragments of (or additions to) the parent molecule having similar functional properties and reactivities as the parent molecule.

Ligation -- refers to the process of forming phosphodiester bonds between two double stranded nucleic acid fragments (Maniatis, T. et al., Id., p. 146). Unless otherwise provided, ligation may be accomplished using known buffers and conditions with T4 DNA ligase.

Milli-Q® water -- water purified through the Milli-Q® filtration system available from Millipore Corporation, Ashby Road, Bedford, MA 01730.

Oligonucleotides -- refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

Plasmid -- a extrachromosomal self-replicating genetic element. Plasmids are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

Reading frame -- the nucleotide sequence from which translation occurs "read" in triplets by the translational apparatus of tRNA and ribosomes and associated factors each triplet corresponding to a particular amino acid. Because each triplet is distinct and of the same length, the coding sequence must be a multiple of three, a base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" being maintained.

Recombinant DNA Cloning Vector -- any autonomously replicating agent, including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- a DNA sequence that controls and allows for autonomous replication of a plasmid or other vector.

RP-HPLC -- an abbreviation for reverse-phase high performance liquid chromatography.

Transcription -- the process whereby information contained in a nucleotide sequence of DNA is transferred to a complementary RNA sequence.

Transfection -- refers to the taking up of an expression vector by a host cell whether or not any coding sequences are in fact expressed. Numerous methods of transfection are known to the ordinarily skilled artisan, for example, CaPO4 and electroporation. Successful transfection is generally recognized when any indication of the operation of this vector occurs within the host cell.

Transformation -- means introducing DNA into an organism so that the DNA is replicable, either as an extrachromosomal element or by chromosomal integration. Transformation may be achieved by the method of Graham, F. and van der Eb, A, (1973) Virology 52:456-7. Other methods such as nuclear injection, protoplast fusion or by calcium treatment using calcium chloride as described by Cohen, S. N. et al., (1972) Proc. Natl. Acad. Sci. (USA) 69:21.

Translation -- the process whereby the genetic information of messenger RNA is used to specify and direct the synthesis of a polypeptide chain.

Vector -- a replicon used for the transformation of cells in gene manipulation bearing polynucleotide sequences corresponding to appropriate protein molecules which when combined with appropriate control sequences confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. The term "vector" as used herein includes Recombinant DNA cloning vectors and Recombinant DNA expression vectors.

A cDNA sequence encoding the pGRF receptor protein including the endogenous signal peptide sequence was isolated from a total porcine mRNA library and comprises the DNA sequence: [Sequence ID#1]

```
            ATG GACAGCGGGG TGTGGGCTGC CTGCATCTTC TGCCTGCTGA
GCTCCCTACC AGTCGCCTTG GGCCACGTGC ACCCGGAATG TGACTTCATC
ACCCAGCTGA GAGAAGACGA GCGAACTTGT CTACAAGCAG CAGACCGGAT
GGCCAACTCC TCCTCGGGCT GTCCTAGGAC CTGGGATGGG CTGTTGTGCT
GgCCGACGGC AGGCCCTGGG GAGTGGGTGA CCCTCCCCTG CCCGGCTTTC
TTCTCTCACT TCAGCTCTGA GCCAGGGGCC CTGAAGCGGG ACTGCACCAC
CACGGGCTGG TCTGAGCCCT TCCCGCCATA TCCCGAGGCT TGCCCTGTGC
CCCTGGAGCT GCTGACTGAT GAGAAATCCT ACTTCTCCAC TGTGAGAATC
GTCTACACCA CGGGCCACAG CGTCTCTGCC GTGGCCCTCT TCGTGGCCAT
CGCCATCCTG GTTGCTCTCA GGAGGCTCCA CTGCCCCAGG AACTACATCC
ACAGCCAGCT GTTCGCCACC TTTATCCTCA AGGCGGGAGC TGTGTTCTTG
AAAGACGCCG CCCTCTTTCA CAGCGAGAAC ACGGACCACT GCAGCTTCTC
CACGGTTCTG TGCAAGGTCT CTGTGGCCAC CTCCCATTTC GCCACCATGA
CCAACTTCAG CTGGCTGCTG GCAGAAGCTG TCTACCTGAC CTGCCTCTTG
GCCTCTACGT CACCCAGCAC GAGGAGGGCC TTCTGGTGGC TGGTTCTCGC
TGGCTGGGGG CTGCCCCTGC TCTTCACTGG CACGTGGGTG GGTTGCAAGT
TGGCCTTTGA GGATGTTGCG TGCTGGGACC TGGACGACAG CTCCCCCTAC
TGGTGGATCA TCAAAGGGCC CATCGTCCTC TCCGTGGGGG TGAACTTTGG
GCTTTTTCTC AATATTATCC GCATCCTGCT GAGGAAACTG GAGCCAGCTC
AGGGCAGCCT CCACACCCAG CCTCAGTACT GGCGTCTCTC CAAGTCAACC
CTTCTCCTCA TCCCGCTGTT TGGAATTCAC TACGTCATCT TCAACTTCCT
GCCTGACAGT GCTGGCCTGG GCATCCGCCT CCCCCTGGAG CTGGGACTGG
GCTCCTTCCA GGGCTTCATT GTTGCCATCC TGTACTGCTT CCTCAACCAA
GAGGTGAGGA CTGAGATCTC ACGGAGGTGG CATGGCCATG ACCCTGAACT
TCTGCCAGCC TGGAGGACTC ATGCCAAATG GGCAAAGCCT TCCCGCTCAA
GGGCGAAGGT CAGTGCGTGT TCACGTGCTG GGTCTTCCCG CCCCCGAGCC
CATGGCGACA CATACCCGGG ACTGGAAGTG CCGGGTCAGT GGTTGTGCCT
TTTCTTGACG TGA
```

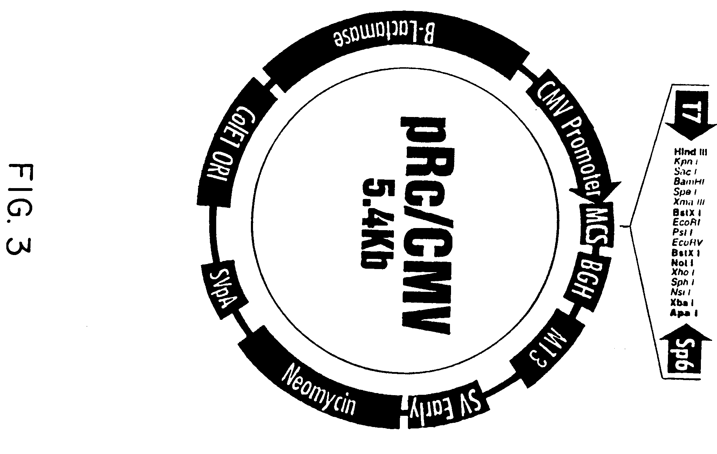

The identity of the protein encoded by the above DNA was demonstrated by recombinant expression of the sequence in a eucaryotic cell and subsequent demonstration of pGRF receptor activity. Briefly, a double stranded counterpart to Sequence ID#1, externally modified to incorporate convenient restriction sites, was integrated into the pRc/CMV vector and recombinantly expressed in 293 cells. The cDNA transcript included the signal peptide region of the native transcript facilitating proper orientation and integration into the transfected cell membrane. Exposure of the transfected 293 cells expressing the protein of Sequence ID#2 on the cell surface demonstrated an intracellular increase in cAMP levels upon exposure to pGRF(1-44)OH. cAMP levels was also measured upon exposure of the transfected cells in response to the addition of secretin and vasoactive intestinal peptide (VIP), two peptides closely related to GRF. The levels of cAMP produced by the exposure of the transfected cells expressing the protein of Sequence ID#2 to VIP and calcitonin demonstrate a selectivity significantly different from the profile produced by exposure to pGRF(1-44)OH indicating that the cDNA encoding the above receptor does indeed constitute a GRF receptor. See data in Tables 1-4 below and in Figure 3 of the attached drawings.

## Table 1.

### Exposure of 293/G5-23 cells to GRF

| [GRF]* | intracellular [cAMP]** |
|---|---|
| $10^{-10}$ | 20 |
| $10^{-9}$ | 60 |
| $10^{-8}$ | 110 |
| $10^{-7}$ | 170 |
| $10^{-6}$ | 270 |
| $10^{-5}$ | 280 |

\*      hormone concentration is expressed in molarity

\*\*      intracellular cAMP is expressed in pmoles/$10^5$ cells

## Table 2.

### Exposure of 293/G5-12 cells to calcitonin

| [calcitonin]* | intracellular [cAMP]** |
|---|---|
| $10^{-10}$ | <10 |
| $10^{-9}$ | 10 |
| $10^{-8}$ | 16 |
| $10^{-7}$ | 26 |
| $10^{-6}$ | 30 |
| $10^{-5}$ | 32 |

\*      hormone concentration is expressed in molarity
\*\*     intracellular cAMP is expressed in pmoles/$10^5$ cells

## Table 3.

### Exposure of 293/G5-12 cells to VIP

| [VIP]* | intracellular [cAMP]** |
|---|---|
| $10^{-10}$ | 28 |
| $10^{-9}$ | 36 |
| $10^{-8}$ | 50 |
| $10^{-7}$ | 64 |
| $10^{-6}$ | 78 |
| $10^{-5}$ | 92 |

\*      hormone concentration is expressed in molarity
\*\*     intracellular cAMP is expressed in pmoles/$10^5$ cells

## Table 4.

### Exposure of 293/G5-12 cells to secretin

| [secretin]* | intracellular [cAMP]** |
|---|---|
| $10^{-10}$ | <10 |
| $10^{-9}$ | <10 |
| $10^{-8}$ | <10 |
| $10^{-7}$ | 10 |
| $10^{-6}$ | 70 |
| $10^{-5}$ | 90 |

\*      hormone concentration is expressed in molarity
\*\*     intracellular cAMP is expressed in pmoles/$10^5$ cells

The invention further provides the pGRF receptor and functional analogs thereof. Functional analogs typically exhibit the same qualitative biological activity as the naturally-occurring analog, although functional analogs also are selected in order to modify the characteristics of pGRF receptor. Functional analogs are ordinarily engineered variations, but such functional analogs include naturally occurring allelic or interspecies variations of the pGRF receptor amino acid sequences.

Examples of such naturally occurring variations are illustrated by the isolation of the G3 isoform of the pGRF Receptor. The G3 pGRF Receptor was isolated from porcine pituitary tissue and comprises the DNA sequence [Sequence ID. #11] and corresponding amino acid sequence [Sequence ID. #12]:

```
ATG GAC AGC GGG GTG TGG GCT GCC TGC ATC TTC TGC CTG CTG AGC TCC
Met Asp Ser Gly Val Trp Ala Ala Cys Ile Phe Cys Leu Leu Ser Ser

CTA CCA GTC GCC TTG GGC CAC GTG CAC CCG GAA TGT GAC TTC ATC ACC
Leu Pro Val Ala Leu Gly His Val His Pro Glu Cys Asp Phe Ile Thr

CAG CTG AGA GAA GAC GAG CGA ACT TGT CTA CAA GCA GCA GAC CGG ATG
Gln Leu Arg Glu Asp Glu Arg Thr Cys Leu Gln Ala Ala Asp Arg Met

GCC AAC TCC TCC TCG GGC TGT CCT AGG ACC TGG GAT GGG CTG TTG TGC
Ala Asn Ser Ser Ser Gly Cys Pro Arg Thr Trp Asp Gly Leu Leu Cys
```

```
TGG CCG ACG GCA GGC CCT GGG GAG TGG GTG ACC CTC CCC TGC CCG GCT
Trp Pro Thr Ala Gly Pro Gly Glu Trp Val Thr Leu Pro Cys Pro Ala

TTC TTC TCT CAC TTC AGC TCT GAG CCA GGG GCC CTG AAG CGG GAC TGC
Phe Phe Ser His Phe Ser Ser Glu Pro Gly Ala Leu Lys Arg Asp Cys

ACC ACC ACG GGC TGG TCT GAG CCC TTC CCG CCA TAT CCC GAG GCT TGC
Thr Thr Thr Gly Trp Ser Glu Pro Phe Pro Pro Tyr Pro Glu Ala Cys

CCT GTG CCC CTG GAG CTG CTG ACT GAT GAG AAA TCC TAC TTC TCC ACT
Pro Val Pro Leu Glu Leu Leu Thr Asp Glu Lys Ser Tyr Phe Ser Thr

GTG AGA ATC GTC TAC ACC ACG GGC CAC AGC GTC TCT GCC GTG GCC CTC
Val Arg Ile Val Tyr Thr Thr Gly His Ser Val Ser Ala Val Ala Leu

TTC GTG GCC ATC GCC ATC CTG GTT GCT CTC AGG AGG CTC CAC TGC CCC
Phe Val Ala Ile Ala Ile Leu Val Ala Leu Arg Arg Leu His Cys Pro

AGG AAC TAC ATC CAC AGC CAG CTG TTC GCC ACC TTT ATC CTC AAG GCG
Arg Asn Tyr Ile His Ser Gln Leu Phe Ala Thr Phe Ile Leu Lys Ala

GGA GCT GTG TTC TTG AAA GAC GCC GCC CTC TTT CAC AGC GAG AAC ACG
Gly Ala Val Phe Leu Lys Asp Ala Ala Leu Phe His Ser Glu Asn Thr

GAC CAC TGC AGC TTC TCC ACG GTT CTG TGC AAG GTC TCT GTG GCC ACC
Asp His Cys Ser Phe Ser Thr Val Leu Cys Lys Val Ser Val Ala Thr

TCC CAT TTC GCC ACC ATG ACC AAC TTC AGC TGG CTG CTG GCA GAA GCT
Ser His Phe Ala Thr Met Thr Asn Phe Ser Trp Leu Leu Ala Glu Ala

GTC TAC CTG ACC TGC CTC TTG GCC TCT ACG TCA CCC AGC ACG AGG AGG
Val Tyr Leu Thr Cys Leu Leu Ala Ser Thr Ser Pro Ser Thr Arg Arg

GCC TTC TGG TGG CTG GTT CTC GCT GGC TGG GGG CTG CCC CTG CTC TTC
Ala Phe Trp Trp Leu Val Leu Ala Gly Trp Gly Leu Pro Leu Leu Phe

ACT GGC ACG TGG GTG GGT TGC AAG TTG GCC TTT GAG GAT GTT GCG TGC
Thr Gly Thr Trp Val Gly Cys Lys Leu Ala Phe Glu Asp Val Ala Cys

TGG GAT CTG GAC GAC AGC TCC CCC TAC TGG TGG ATC ATC AAA GGG CCC
Trp Asp Leu Asp Asp Ser Ser Pro Tyr Trp Trp Ile Ile Lys Gly Pro

ATC GTC CTC TCC GTG GGG GTG AAC TTT GGG CTT TTT CTC AAT ATT ATC
Ile Val Leu Ser Val Gly Val Asn Phe Gly Leu Phe Leu Asn Ile Ile

CGC ATC CTG CTG AGG AAA CTG GAG CCA GCT CAG GGC AGC CTC CAC ACC
Arg Ile Leu Leu Arg Lys Leu Glu Pro Ala Gln Gly Ser Leu His Thr

CAG CCT CAG TAC TGG CGT CTC TCC AAG TCA ACC CTT CTC CTC ATC CCA
Gln Pro Gln Tyr Trp Arg Leu Ser Lys Ser Thr Leu Leu Leu Ile Pro

CTG TTT GGA ATC CAC TAC GTC ATC TTC AAC TTC CTG CCT GAC AGT GCT
Leu Phe Gly Ile His Tyr Val Ile Phe Asn Phe Leu Pro Asp Ser Ala
```

```
GGC CTG GGC ATC CGC CTC CCC CTG GAG CTG GGA CTG GGC TCC TTC CAG
Gly Leu Gly Ile Arg Leu Pro Leu Glu Leu Gly Leu Gly Ser Phe Gln

GGC TTC ATT GTT GCC ATC CTG TAC TGC TTC CTC AAC CAA GAG GTG AGG
Gly Phe Ile Val Ala Ile Leu Tyr Cys Phe Leu Asn Gln Glu Val Arg

ACT GAG ATC TCA CGG AGG TGG CAT GGC CAT GAC CCT GAA CTT CTG CCA
Thr Glu Ile Ser Arg Arg Trp His Gly His Asp Pro Glu Leu Leu Pro

GCC TGG AGG ACT CAT GCC AAA TGG GCA AAG CCT TCC CGC TCA AGG GCG
Ala Trp Arg Thr His Ala Lys Trp Ala Lys Pro Ser Arg Ser Arg Ala

AAG GTG CTG ACA ACT GTG TGC TAA
Lys Val Leu Thr Thr Val Cys
```

The G3 isoform of the pGRF Receptor (Sequence ID#12) demonstrates a greater stimulation of intracellular cAMP in response to GRF binding as evidenced by a comparison of the following Table 5 with Table 1 above.

## Table 5.

### Exposure of 293/G3-23 cells to GRF

| [GRF]* | intracellular [cAMP]** |
|---|---|
| $10^{-11}$ | 20 |
| $10^{-10}$ | 150 |
| $10^{-9}$ | 250 |
| $10^{-8}$ | 300 |
| $10^{-7}$ | 325 |
| $10^{-6}$ | 350 |

\*     hormone concentration is expressed in molarity
\*\*   intracellular cAMP is expressed in pmoles/$10^5$ cells

The G3 isoform of the pGRF receptor provides an example of a functional analog of the pGRF receptor contemplated by the present invention.

Functional analogs are ordinarily prepared by modification of the DNA encoding the pGRF receptor and thereafter expressing the DNA in recombinant cell culture. Techniques for making substitutional mutations at predetermined sites in DNA having a known sequence are well known, for example M13 primer mutagenesis. The mutations that might be made in the DNA encoding the functional analog pGRF receptor must not place the sequence out of reading frame and preferably will not create complementary regions that could produce secondary mRNA structure (EP 75,444A).

Functional analogs of the pGRF receptor are generally created by modification of the amino acid sequence of the protein in a specific and limited manner. While the site for introducing an amino acid sequence variation is predetermined, the mutation per se need not be predetermined. For example, in order to optimize the, performance of a mutation at a given site, random mutagenesis may be conducted at the target codon or region and the expressed pGRF receptor functional analogs screened for the optimal combination of desired activity.

Functional analogs of the pGRF receptor are typically generated by deletion, insertion, or substitutions of a single (or few) amino acid residues. Such modifications generally are made in accordance with the following Table 6 to produce functional analogs of the pGRF receptor.

TABLE 6

| Original Residue | Exemplary Substitutions |
|---|---|
| Ala | ser |
| Arg | lys |
| Asn | gln; his |
| Asp | glu |
| Cys | ser |
| Gln | asn |
| Glu | asp |
| Gly | pro |
| His | asn gln |
| Ile | leu., val |
| Leu | ile; val |
| Lys | arg; gln; ,glu |
| Mel | leu ile |
| Phe | met leu; tyr |
| Ser | thr |
| Thr | ser |
| Trp | tyr |
| Tyr | trp; phe |
| Val | ile; leu |

Substantial changes in function or immunological identity are made by selecting substitutions that are less conservative than those in Table 6, i.e., selecting residues that differ more significantly in their effect on maintaining (a) secondary or tertiary structure of the polypeptide backbone, (b) the charge or hydrophobicity of the residue, or (c) the bulk of the side chain. The substitutions which in general are expected to produce the greatest changes in pGRF receptor properties will be those in which (a) a hydrophilic residue is substituted by a hydrophobic residue; (b) a cysteine or proline is substituted for any other residue; (c) a residue having a positive charged side chain is substituted for a negatively residue; or (d) a residue having a bulky side chain is substituted for one not having a side chain.

Site specific mutagenesis may also be employed to eliminate N- or O-linked glycosylation sites resulting in non-glycosylated functional analogs of the pGRF receptor. The non-glycosylated pGRF receptor may be recombinantly produced in prokaryotic cell culture. Deletions of cysteine or other labile residues also may be desirable, for example in increasing the oxidative stability of the pGRF receptor. Deletions or substitutions of potential proteolysis sites, e.g. Arg-Arg, may also be accomplished by deleting one of the basic residues or substituting one by glutaminyl or histidinyl residues.

A preferred pGRF receptor encoded by Sequence ID#1 and comprising the endogenous signal peptide possesses the amino acid sequence: [Sequence ID#2]

```
MDSGVWAACI  FCLLSSLPVA  LGHVHPECDF  ITQLREDERT  CLQAADRMAN
SSSGCPRTWD  GLLCWPTAGP  GEWVTLPCPA  FFSHFSSEPG  ALKRDCTTTG
WSEPFPPYPE  ACPVPLELLT  DEKSYFSTVR  IVYTTGHSVS  AVALFVAIAI
LVALRRLHCP  RNYIHSQLFA  TFILKAGAVF  LKDAALFHSE  NTDHCSFSTV
LCKVSVATSH  FATMTNFSWL  LAEAVYLTCL  LASTSPSTRR  AFWWLVLAGW
GLPLLFTGTW  VGCKLAFEDV  ACWDLDDSSP  YWWIIKGPIV  LSVGVNFGLF
LNIIRILLRK  LEPAQGSLHT  QPQYWRLSKS  TLLLIPLFGI  HYVIFNFLPD
SAGLGIRLPL  ELGLGSFQGF  IVAILYCFLN  QEVRTEISRR  WHGHDPELLP
AWRTHAKWAK  PSRSRAKVSA  CSRAGSSRPR  AHGDTYPGLE  VPGQWLCLFL
T
```

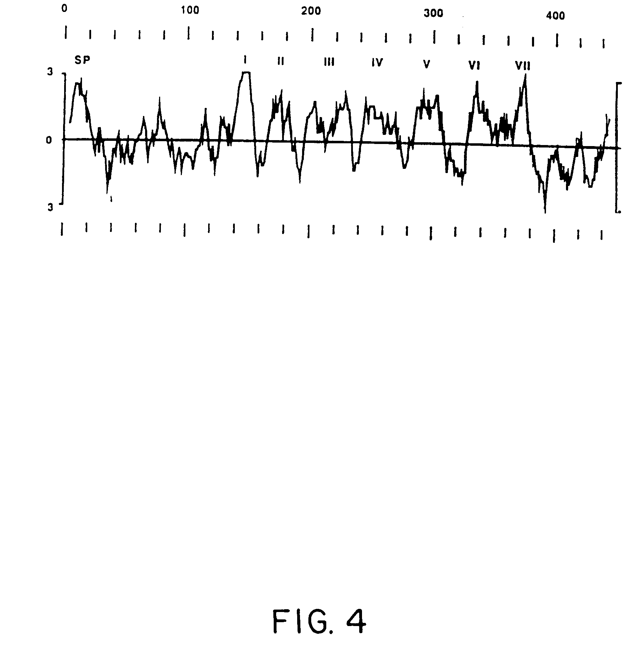

A hydrophobicity plot of the amino acid sequence of this porcine GRF receptor is shown in Figure 4 of the attached drawings. The amino terminal portion of the porcine GRF receptor from the amino terminus to approximately amino acid residue No. 22 constitutes the putative signal peptide sequence. The portion of the pGRF receptor from approximately amino acid residue No. 23 to approximately amino acid No. 140 constitutes the putative extracellular domain of the GRF receptor. From approximately amino acid No. 141 to No. 380 constitutes the 7 hydrophobic transmembrane domains. Leftkowitz has performed site-directed mutagenesis with βdrenergic receptor and his results showed that the third intracellular domain (approximately amino acid 310 to 330) is important in coupling to the G-protein (Taylor, C.N. (1990) Biochem. J. 272:1-13).

The pGRF receptor cDNA is characterized by the presence of a DNA sequence encoding a signal (or leader) polypeptide which serves to direct the ultimate location of the protein in the cell membrane. Generally, signal peptides are proteolytically cleaved from a residual protein as part of the secretory process in which the protein is transported into the host cell periplasm or culture medium. However, the functional GRF receptor may include the signal peptide in its primary sequence yet retain GRF binding activity. Analogs of the pGRF receptor incorporating modified signal peptides are within the scope of the present invention.

It is well known in the art that signal peptides facilitate the extracellular discharge of secretory proteins in both prokaryotic and eukaryotic environments. It has been shown that the addition of a heterologous signal peptide to a normally cytosolic protein will result in the extracellular transport of the normally cytosolic protein in E. coli. MacIntyre, et al., (1987) J.Biol.Chem. 262:8416-8422. It is well known in the art that alternate signal peptide sequences may function with heterologous coding sequences. For instance, a DNA sequence encoding the signal peptide from another receptor such as the secretin receptor may be substituted for the DNA sequence encoding the signal peptide of the pGRF receptor resulting in a heterologous protein retaining pGRF receptor characteristics. The recombinant production of such fusion proteins maybe accomplished by the addition of a DNA sequence encoding a signal peptide appropriate to the host organism inserted 5' to, and in reading frame with, the protein coding sequence.

Signal peptides are well known in the art which could be similarly incorporated into the pGRF structure to make other functional analogs of the pGRF receptor. The signal peptide may be microbial or mammalian, but is preferably is mammalian. In the preferred practice of the invention the signal peptide used is a signal peptide native to a secretory protein of the host cell line. In the most preferred practice of the invention as exemplified herein, the signal peptide is the native 22 amino acid pGRF receptor presequence.

Furthermore, the signal sequence may be wholly synthetic. Synthetic "idealized" signal peptides have been shown to function in both prokaryotic and eukaryotic environments. von Heijne, G. (1990) J. Membrane Biol. 115: 195-201. The principles of signal peptides are similar in both prokaryotic and eukaryotic organisms. Both prokaryotic and eukaryotic signal peptides possess an overall three domain structure and with no precise sequence conservation necessary to preserve function. von Heijne, G., supra. Generally, the presence of basic and/or charged amino acid residues near the amino terminus of the structural protein inhibits secretion. Yamane, K., et al. (1988) J.Biol.Chem. 263:19690-19696, Summers, R.G., et al. (1989) J.Biol.Chem. 264:20082-20088. In order to insure the efficient cleavage of the signal peptide from the fusion protein construct, it is desirable to maintain the nature of the amino acid sequence at the interface between the signal peptide and the coding sequence of the mature art protein. Conservation of charge and hydrophobicity and the elimination of charged residues immediately downstream of the signal peptide cleavage point are generally important to efficient translocation. However, it is not critical that any one particular amino acid sequence be maintained.

The invention further provides transgenic animals produced using the pGRF coding sequence or DNA sequences encoding functional analogs of the pGRF useful as research tools in the study of GRF activity. Construction of transgenic animals which express heterologous foreign proteins is well known in the art. See Wagner, et al (U.S. Patent No. 4,873,191 issued October 10, 1989), Evans, et al. (U.S. Patent No. 4,870,009 issued September 26, 1989) the entire teachings of which are hereby incorporated by reference, Cline, et al.

(1980) Nature <u>284</u>:422-425, and Capecchi M. R. (1980) Cell <u>22</u>:479:488.

The invention also provides methods of making pGRF receptor. The compounds of the present invention may be produced either by recombinant DNA technology or well known chemical procedures, such as solution or solid-phase peptide synthesis, or semi-synthesis in solution beginning with protein fragments coupled through conventional solution methods.

The synthesis of the pGRF receptor or of pGRF peptide fragments thereof may proceed by solid phase peptide synthesis or by recombinant methods. The principles of solid phase chemical synthesis of polypeptides are well known in the art and may be found in general texts in the area such as Dugas, H. and Penney, C., <u>Bioorganic Chemistry</u> (1981) Springer-Verlag, New York, pgs. 54-92. For examples, peptides may be synthesized by solid-phase methodology utilizing an Applied Biosystems 430A peptide synthesizer (commercially available from Applied Biosystems, Foster City California) and synthesis cycles supplied by Applied Biosystems. Boc amino acids and other reagents are commercially available from Applied Biosystems and other chemical supply houses. Sequential Boc chemistry using double couple protocols are applied to the starting p-methyl benzhydryl amine resins for the production of C-terminal carboxamides. For the production of C-terminal acids , the corresponding PAM resin is used. Asparagine, Glutamine, and Arginine are coupled using preformed hydroxy benzotriazole esters. The following side chain protection may be used:

Arg, Tosyl
Asp, cyclohexyl
Glu, cyclohexyl
Ser, Benzyl
Thr, Benzyl
Tyr, 4-bromo carbobenzoxy

Boc deprotection may be accomplished with trifluoroacetic acid (TFA) in methylene chloride. Following completion of the synthesis the peptides may be deprotected and cleaved from the resin with anhydrous hydrogen fluoride containing 10% meta-cresol. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether, and the peptide extracted with glacial acetic acid and lyophilized. Purification is accomplished by size-exclusion chromatography on a Sephadex G-10 (Pharmacia) column in 10% HOAc.

The pGRF receptor may also be produced by recombinant methods. Recombinant methods are preferred if a high yield is desired. The basic steps in the recombinant production of pGRF receptor include:

a) construction of a synthetic or semi-synthetic (or isolation from natural sources) a DNA encoding the pGRF receptor,

b) integrating the coding sequence into an expression vector in a manner suitable for the expression of the pGRF receptor either alone or as a fusion protein,

c) transforming an appropriate eukaryotic or prokaryotic host cell with the expression vector, and

d) recovering and purifying the recombinantly produced pGRF receptor.

As previously stated, the coding sequence may be wholly synthetic, semi-synthetic or the result of modification of the native pGRF receptor cDNA. The pGRF receptor cDNA isolated in substantial accordance with the teaching of the examples herein comprises the DNA sequence of Sequence ID#1.

Synthetic genes, the <u>in</u> <u>vitro</u> or <u>in</u> <u>vivo</u> transcription and translation of which will result in the production of pGRF receptor may be constructed by techniques well known in the art. Owing to the natural degeneracy of the genetic code, the skilled artisan will recognize that a sizable yet definite number of DNA sequences may be constructed which encode pGRF receptor. In the preferred practice of the invention, synthesis of the pGRF receptor is achieved by recombinant DNA technology.

The gene encoding the pGRF receptor may be created by synthetic methodology. Such methodology of synthetic gene construction is well known in the art. Brown, <u>et al.</u> (1979) Methods in Enzymology, Academic Press, N.Y., Vol. <u>68</u>, pgs. 109-151. The DNA sequence corresponding to the synthetic pGRF receptor gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404).

It may desirable in some applications to modify the coding sequence of the pGRF receptor relative to the native protein so as to incorporate a convenient protease sensitive cleavage site, e.g., between the signal peptide and the structural protein facilitating the controlled excision of the signal peptide from the fusion protein construct.

The pGRF receptor may be made either by direct expression or as fusion protein comprising the pGRF receptor followed by enzymatic or chemical cleavage. A variety of peptidases (e.g. trypsin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., Carter P. Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

To effect the translation of the desired pGRF receptor or analog, one inserts the engineered synthetic DNA sequence in any of a plethora of appropriate recombinant DNA expression vectors through the use of appropriate restriction endonucleases. The pGRF receptor is a relatively large protein. A synthetic pGRF receptor coding sequence is designed to possess restriction endonuclease cleavage sites at either end of the transcript to facilitate isolation from and integration into these expression and amplification and expression plasmids. The isolated cDNA GRF receptor coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The choice of restriction sites are chosen so as to properly orient the pGRF receptor coding sequence with control sequences to achieve proper in-frame reading and expression of the pGRF receptor.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, E. coli is typically transformed using pBR322, a plasmid derived from an E. coli species (Bolivar, et al., Gene 2: 95 [1977]), pBR322 contains genes for ampicillin and tetracycline resistance and Thus provides easy means for identifying transformed cells. The pBR322 plasmid, or other microbial plasmid must also contain or be modified to contain promoters and other control elements commonly used in recombinant DNA construction.

The pGRF receptor coding sequence must be positioned so as to be in proper reading frame with the promoter and ribosome binding site of the expression vector, both of which are functional in the host cell in which the pGRF receptor is to be expressed. In the preferred practice of the invention, the promoter-operator region is placed in the same sequential orientation with respect to the ATG start codon of DNA sequence encoding the protein of Sequence ID#2 as the promoter-operator occupies with respect to the ATG-start codon of the gene from which it was derived. Synthetic or modified promoter-operator regions such as the tac promoter are well known in the art. When employing such synthetic or modified promoter-operator regions they should be oriented with respect to the ATG-start codon of the pGRF receptor coding sequence as directed by their creators.

The pGRF receptor or analogs may be recombinantly produced in eukaryotic expression systems. General principles involved in the recombinant expression of receptors is described in "Expression of receptor genes in cultured cells" Ch. 11 of Receptor Biochemistry: A Practical Approach, edited by E.C. Hulme IRL Press, Oxford University Press, Walton St. Oxford OX2 6DP, England.

Preferred promoters controlling transcription in mammalian host cells may be obtained from various sources, for example, the genomes of viruses such as: polyoma. Simian Virus 40 (SV40), adenovirus, retroviruses, hepatitis-B virus and most preferably cytomegalovirus, or from heterologous mammalian promoters, e.g. βactin promoter. The early and late promoters of the SV40 virus are conveniently obtained as an SV40 restriction fragment which also contains the SV40 viral origin of replication. Fiers, et al., (1978) Nature, 273:113. The entire SV40 genome may be obtained from plasmid pBRSV, ATCC 45019. The immediate early promoter of the human cytomegalovirus may be obtained from plasmid pCMBβ (ATCC 77177). Of course, promoters from the host cell or related species also are useful herein.

Transcription of a DNA encoding pGRF receptor by higher eukaryotes is increased by inserting an enhancer sequence into the vector. Enhancers are cis-acting elements of DNA, usually about 10-300 bp, that act on a promoter to increase its transcription. Enhancers are relatively orientation and position independent having been found 5' (Laimins, L. et al., [1981] PNAS 78:993) and 3' (Lusky, M. L., et al., [1983] Mol. Cell Bio. 3:1108) to the transcription unit, within an intron (Banerji, J. L. et al., [1983] Cell 33:729) as well as within the coding sequence itself (Osborne, T. F., et al., [1984] Mol. Cell Bio. 4:1293). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding pGRF receptor. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BgIII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK⁻) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., (1982) J. Molec. Appl. Genet. 1: 327, mycophenolic acid, Mulligan, R. C. and Berg, P. (1980) Science 209:1422 or hygromycin, Sugden, B. et al., (1985) Mol Cell. Biol. 5:410-413. The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression of the GRF receptor is pRc/CMV. A restriction site and function map of pRc/CMV appears in Figure 3 of the accompanying drawings. pRc/CMV is commercially available from Invitrogen Corporation, 3985 Sorrento Valley Blvd., San Diego, CA 92121.

To confirm correct sequences in plasmids constructed, the ligation mixtures are used to transform E. coli K12 strain DH5α (ATCC 31446) and successful transformants selected by antibiotic resistance where appropriate. Plasmids from the transformants are prepared, analyzed by restriction and/or sequence by the method of Messing, et al., (1981) Nucleic Acids Res. 9:309.

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al. (1989) Molecular Cloning: A Laboratory Manual, Cold Spring Harbor Press, Cold Spring Harbor Laboratory, Cold Spring Harbor, New York or Current Protocols in Molecular Biology (1989) and supplements.

Preferred suitable host cells for expressing the vectors of this invention encoding pGRF receptor in higher eukaryotes include: African green monkey kidney line cell line transformed by SV40 (COS-7, ATCC CRL-1651); transformed human primary embryonal kidney cell line 293,( Graham, F. L. et al. (1977) J. Gen Virol. 36:59-72, Virology 77:319-329, Virology 86:10-21); baby hamster kidney cells (BHK-21(C-13), ATCC CCL-10, Virology (1962) 16:147); chinese hamster ovary cells CHO-DHFR⁻ (ATCC CRL-9096), mouse Sertoli cells (TM4, ATCC CRL-1715, Biol. Reprod. (1980) 23:243-250); african green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., (1979) Nature 282:39; Kingsman et al., [1979] Gene 7:141 ; Tschemper et al., [1980] Gene 10:157) is commonly used. This plasmid already contains the trp gene which provides a selection marker for a mutant strain of yeast lacking the ability to grow in tryptophan, for example ATCC no. 44076 or PEP4-1 (Jones, [1977], Genetics 85:12).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjuction with the CYC1 promoter on plasmid YEpsec--hl1beta ATCC 67024), also are advantageously used with yeast promoters.

Prokaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the βlactamase (vector pGX2907 [ATCC 39344] contains the replicon and βlactamase gene) and lactose promoter systems (Chang et al., [1978] Nature, 275:615; and Goeddel et al., [1979] Nature 281:544), alkaline phosphatase, the tryptophan (trp) promoter system (vector pATH1 [ATCC 37695] is designed to faciliatate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding pGRF receptor using linkers or adaptors to supply any required restriction sites. Promoters for use in bacterial systems also will contain a Shine-Dalgarno sequence operably linked to the DNA encoding pGRF receptor.

PGRF receptor includes amino acid sequence mutants, glycosylation variants and covalent or aggregative conjugates with other chemical moieties. These GRF receptor, functional derivatives, as well as glycosylation variants and covalent or aggregative conjugates with other chemical moieties may be used according to conventional techniques for the construction and isolation of antibodies against these molecules. See e.g., Kohler and Milstein (1975) Nature 256:497. PGRF receptor include covalent derivatives prepared by linkage of functionalities to groups which are found in the pGRF receptor amino acid side chains or at the N, or C-termini, by means known in the art. These derivatives may, for example, include: aliphatic esters or amides of the carboxyl terminus or residues containing carboxyl side chains, O-acyl derivatives of hydroxyl group-containing residues, and N-acyl derivatives of the amino terminal amino acid or amino- group containing residues, e.g. Lysine or arginine. Acyl groups are selected from the group of alkyl-moieties (including C3 to C18 normal alkyl), thereby forming alkaloyl aroyl species.

A major group of derivatives are covalent conjugates of pGRF receptor or their fragments with other proteins or polypeptides. These derivatives are synthesized in recombinant culture as N, or C-terminal fusions or by the use of bifunctional agents known per se for use in cross- linking proteins to insoluble matrices through reactive side-groups. Preferred GRF receptor derivatization sites with cross-linking agents are at cysteine and lysine residues. Preferred agents are N-maleimidobenzoyl succinimide ester and N-hydroxysuccinimide.

Covalent or aggregative derivatives are useful as immunogens, reagents in immunoassay or for affinity purification procedures of pGRF or other binding ligands. For example, pGRF receptor is insolubilized by covalent bonding to cyanogen bromide-activated Sepharose by methods known per se or adsorbed to polyolefin surfaces (with or without glutaraldehyde cross-linking) for use in the assay or purification of anti-pGRF receptor antibodies or pGRF. pGRF receptor also is labelled with a detectable group, e.g., radioiodinated by the chloramine T procedure, covalently bound to rare earth chelates or conjugated to another fluorescent moiety for use in diagnostic assays.

The ability of GRF to bind to the GRF receptor is essential in the cascade to allow the release of growth hormone. In developing agents which either inhibit or facilitate the release of GH, it therefore would be desirable to determine those agents which interfere with the formation of the [GRF/pGRF receptor] complex. The instant invention provides such a screening system useful for discovering agents which compete with pGRF for binding to the pGRF receptor, said screening system comprising the steps of:

a) recombinantly producing a pGRF receptor,

b) exposing said pGRF receptor to a potential inhibitor of the [pGRF-pGRF receptor] complex,

c) introducing GRF,

d) removing non-specifically bound molecules, and

e) quantifying the concentration of bound potential inhibitor and/or pGRF.

This allows one to rapidly screen for inhibitors of the formation of the [GRF/pGRF receptor] complex. Utilization of the screening system described above provides a sensitive and rapid means to determine compounds which interfere with the formation of the [GRF/GRF receptor] complex. This screening system may also be adapted to automated procedures such as a Pandex® (Baxter-Dade Diagnostics) system allowing for efficient high-volume screening for potential therapeutic agents.

The procedure of such a screening protocol proceeds as follows. A pGRF receptor is prepared as elsewhere described herein, preferably using recombinant DNA technology. A sample of a test compound is then introduced to the reaction vessel containing the pGRF receptor followed by the addition of GRF. Unbound molecules are washed free and the eluent inspected for the presence of GRF or the test compound.

For example, in a preferred method of the invention, radioactively or fluorescently labelled GRF may be used. The eluent is then scored for fluorescence or radioactivity. The absence or diminution of fluorescence or radioactivity indicates the formation of the GRF/pGRF receptor complex. This indicates that the test compound has not disrupted the formation of the GRF/pGRF Receptor complex. The presence of fluorescence or radioactivity indicates that the test compound has disrupted the formation of the GRF/pGRF receptor complex. Similarly, a radioactively or fluorescently labelled test compound may be used in which case the same steps as outined above would be used except that the interpretation of results would be the converse of using radioactively or fluorescently labelled GRF.

The pGRF receptor may be free in solution or bound to a solid support. In the preferred practice of the invention, the pGRF receptor is bound to a solid support. Examples of such solid supports include insoluble natural or synthetic polymers, such as a porous membrane, bead, microparticle, chromatographic resin, microtiter plate, mica, and the like. A variety of cross-linking methods, such as through the side chains of lysine residues or other various chemical means, are well known in the art and may be used for immobilizing the pGRF receptor to a solid supports.

The previously described screening system identifies compounds which competitively bind to the pGRF receptor. Determination of the ability of such compounds to stimulate or inhibit the action of the pGRF receptor is essential to further development of such compounds for therapeutic applications. The need for an bioactivity assay system which determines the response of the pGRF receptor to a compound is clear. The instant invention provides such a bioactivity assay, said assay comprising the steps of:

a) transfecting a mammalian host cell with an expression vector comprising a DNA encoding a pGRF receptor and a signal peptide,

b) culturing said host cell under conditions such that the DNA encoding the pGRF receptor and signal peptide are expressed,

c) exposing said host cell so transfected to a test compound, and

d) quantifying the intracellular cAMP level in response to said test compound.

In the preferred practice of the invention as exemplified herein, a stable 293 cell line containing the pRC/CMV-G5 (as prepared in accordance with the Examples herein) was grown to confluency in 24-well plates. The cells were then incubated at 37°C for approximately 45 min in a solution containing various concentrations of test compounds ($10^{-5}$ to $10^{-10}$ M). After removing the solution, 0.5 ml of 60% ethanol was added to lyse the cells in each well. An aliquot was taken, dried in vacuo, and the level of cAMP was determined. Intracellular cAMP levels were determined using the procedure decribed by Ishihara et al., (1991) using a cyclic AMP assay kit (Cat. no. TRK.432), commercially available from Amersham Corporation 2636 South Clearbrook Drive, Arlington Heights, IL 60005.

## EXAMPLES

The following examples are provided to further illustrate the invention described herein but are not intended to be limitations thereof.

## EXAMPLE 1. ISOLATION OF TOTAL RNA FROM PORCINE PITUITARY TISSUES

The following procedure was adapted from the procedure as disclosed in Sambrook, J., Fritch, E.F., and Maniatis, T. (1989) Molecular Cloning, vol 1, p. 7.19 - 7.22. Briefly, 25 ml of 4M Guanidine isothiocyanate buffer [16.7 mM sodium citrate, 0.5% sodium lauryl sarcosine, 0.17% antifoam A and 0.7% beta-mercaptoethanol, pH 7.0] were added to 3.5 g of pig pituitary (commercially available from Pel-Freez Inc., AR) in a 50 ml sterile Falcon tube. The tissue was homogenized with a tissuemizer at high speed for 1-2 min. The homogenized tissue was centrifuged at 5000xg for 5 min at room temperature.

The supernatant was layered onto a 9.5 ml cushion of 5.7 M CsC1, 16.6 mM sodium acetate in a Beckman centrifuge tube for SW28 rotor. The material was centrifuged at 25°C for 24 hrs at 25,000 rpm. After removing supernatant, drain out liquid and cut off the bottom of the tube. The pellet was rinsed with 70% ethanol at room temperature and dried at room temperature.

The pellet of RNA was redissolved in sterile Milli-Q® water. The RNA was purified by phenol extraction, and then precipitated by the addition of ethanol. The pellet was rinsed with 70% ethanol and dried *in vacuo*. Approximately 4.9 mg of total RNA was isolated from 3.5 g of pig pituitary tissues.

## EXAMPLE 2. cDNA SYNTHESIS FROM TOTAL RNA

The following procedure was performed using a BRL kit (Catalog No. 80255A/SB) in substantial accordance with the instructions provided by the manufacturer. Briefly, porcine pituitary total RNA (1 mg/ml, 1 μl prepared in accordance with Example 1 above) was mixed with 1 μl of 50 μM random hexamer primer (commercially available from Perkin Elmer-Cetus as part of the GeneAmp® PCR Kit No.N808-0017) and 10 μl Milli-Q® water in a 1.5 ml Eppendorf tube. The mixture was heated to 70°C for 10 min, chilled on ice for 2 min , and spun down in a microcentrifuge for one second. Four μl of 5x reaction buffer (250 mM Tris-HCl, pH 8.3, 375 mM KCl, 15 mM $MgCl_2$), 2 μl of 0.1 M DTT, 1 μl of dNTP stock (10 mM each of dATP, dGTP, dCTP, and dTTP at pH 7.0) was then added to the sample and the mixture was incubated at 37°C for 2 min. One μl of M-MLV-reverse transcriptase (200 U/μl, BRL) was added and the reaction mixture was incubated at 37°C for 1 hour. The mixture was heated at 95°C for 5 minutes, cooled on ice for 5 minutes and spun for 1 second. This random primed cDNA reaction mixture was then used for PCR amplification in accordance with the teaching of Example 3 below.

## EXAMPLE 3. PCR AMPLIFICATION

The following procedure was performed using a Perkin Elmer-Cetus Gene Amp PCR Reagent Kit (commercially available from The Perkin Elmer-Cetus Corporation, 761 Main Avenue, Norwalk CT 06859-0156) in substantial accordance with the teaching of the instructions provided therewith. Briefly, in a sterile 0.5 ml tube, add 10 μl of a 10 x reaction buffer, 16 μl of dNTP mixture (each at 1.25 mM), 25 pmoles of up-stream primer, 25 pmoles of down-stream primer, 1 μl of the cDNA reaction mixture or 1 μl of 1:10 dilution of the cDNA reaction mixture. Sterile Milli-Q® water was added to achieve a final volume of 99 μl. One μl of diluted (1:2 or 1:3) AmpliTAQ DNA polymerase (5 U/μ commercially available from Perkin Elmer-Cetus) then added. The reaction mixture was mixed and one drop (about 50 μ) of mineral oil (commercially available from Sigma Chemical Co.) was added. PCR was carried out for 30 cycles in 3 steps:

(a) denaturation step, 30 seconds at 95°C;

(b) annealing step, 1 min at 5°C-10°C below $T_m$ of primers;

(c) polymerization step, 1 min at 72°C.

10 μl of the PCR reaction mixture was withdrawn after reaction was completed and electrophoresed on a 6% acrylamide gel or 0.7 % agarose gel for analysis. The analyzed PCR products showed the following oligonucleotide pairs gave a 700bp cDNA fragment.

Degenerate oligonucleotide primers:

5440 (downstream) (Sequence ID#9)

    5'-CTGCACCTCACCATTGAGGAAGCAGTA-3'

5441 (upstream (Sequence ID#10)

    5'-TTCCGGAGGCTGCAYTGCACYCGMAACTACAT 3'

wherein Y= C or T and M= A or C

## EXAMPLE 4. CLONING OF PCR PRODUCTS

### EXAMPLE 4.a. Amplification of the 700bp Fragment by PCR.

The aforementioned 700bp cDNA fragment was purified on 6% polyacrylamide gel as above. The approximately 700 base pairs gel band was excised and soaked in sterile Milli-Q® water (approximately 500 μl) for approximately 5 hours or overnight at room temperature. Using 3 μl of soaking solution as a template, the same (5440 and 5441) oligonucleotides as primers, another PCR amplification was run under the same PCR conditions as described above. The second PCR products were electrophoresed on a 6% polyacrylamide gel. DNA bands corresponding to approximately 700 base pairs were excised from the gel. The DNA was electroeluted from the gels (1x TBE, 50 volts/overnight using equipment commercially available from Epigene). After precipitation of DNA, the DNA pellet was resuspended in 50 μl of Milli-Q® water.

### EXAMPLE 4.b. Prepare blunt-end DNA fragments

5 μl of 10x Kinase buffer (500 mM tris-HCl, pH 7.5, 100 mM $MgCl_2$, 100 mM DTT), 5 μl of 10 mM ATP, 5 μl of 2.5 mM dNTPs, 10 μl of T4 DNA polymerase (1U/μl, commercially available from Pharmacia),1 μl of T4 DNA kinase (10 U/μl, Pharmacia) and 4 μl of sterile water and 20 μl of the purified cDNA fragment (700 bp) were combined in a 1.5 ml Eppendorf tube. The reaction mixture was incubated at 37°C for 1 hour. The enzymes were then inactivated by incubation at 68°C for 10 min.

### EXAMPLE 4.c. Ligation; transformation

pUC18 DNA (0.1 micrograms linearized with Smal) was ligated to blunt-ended PCR fragments (0.06 μg, 700 basepairs) in the presence of 3 μl of 10x Kinase buffer, 1.5 μl of 10 mM ATP, 3 μl of sterile water and 1 μl of T4 DNA Ligase (BRL) at 14°C overnight in a final volume of 20 μl. The ligation mixture was diluted with TE buffer (pH 7.4) to 1:5. 5 μl of the diluted ligation mixture was used to transform 80 μl DH5α competent cells (commercially available from BRL).

After incubation on ice for one hour, the cells were heat shocked at 42°C for 45 seconds and then chilled on ice for 10 min. 800 μl S.O.C. medium (commercially available from BRL) was added and the reaction mixture was incubated for another 1 hour at 37°C. Cells were plated on TY agar containing 2% Xgal, 50 μM IPTG and 100 μg/ml ampicillin (Ap) and then incubated at 37°C overnight.

### EXAMPLE 4.d. Screen transformants by PCR

12 white colonies from the plates were picked and used to inoculate 3 ml TY medium containing 100 μg/ml Ap and cultured at 37°C for 4 hours. 5 microliters of culture was added to a solution containing 5 μl of 10x PCR buffer, 8 μ of 1.25 mM dNTPs, 0.5 μl of 1% gelatin, 0.5 μl of 10% Triton x-100, 1 μl of 15 μM universal forward primer No. 2977 ($T_m$ = 50°C) having DNA sequence:

```
5'-GTAAAACGACGGCCAGT-3'    (Sequence ID#3)
```

1 μl of 15 μM universal reverse primer (No. 2976, $T_m$ = 50°C) having DNA sequence:

```
5'-CAGGAAACAGCTATGAC-3'    (Sequence ID#4)
```

and 28 μl of sterile water. The mixture was heated to 95°C for 15 min and quickly cooled on ice. One μl of TAQ DNA polymerase (2.5 U/μl) (commercially available from Perkin Elmer Cetus) and 50 μl of mineral oil was added to the reaction mixture. PCR was run for 30 cycles: 95°C, 1/2 min.; 40°C, 1 min.; 72°C, 1 min.

A 10 μl sample of the resulting reaction mixture was electrophoresed on a 6% polyacrylamide gel. The colonies producing a band of about 700 bp size were selected as candidates and were subjected to sequencing analysis. Two clones were identified as G and S based on sequence homology with other G-protein coupled receptors.

## EXAMPLE 5. PREPARATION OF 32P-DNA PROBE USING PCR

PCR was used to prepare 700 bp cDNA hybridization probes to screen porcine pituitary cDNA library to isolate the pGRF Receptor cDNA.

The [32P]-cDNA probes were prepared in the following manner. The reaction mixture containing 4 μl of 10x PCR buffer, 3 μl of a mixture of dATP, dGTP and dTTP (0.2 mM each), 2 μl of template plasmid DNA (5 nanograms/microliter), 40 picomoles of each primer, 20 μl [32P]-α-dCTP (3000Ci/mmole), 1 microliter of TAQ DNA polymerase (5 U/μl) and sterile water was added to adjust the total volume to 40 μl. After PCR amplification was done, purified [32P]-DNA probe was isolated using a Quick-spin column (G-50 Sephadex®, commercially available from Boehringer Mannheim) to eliminate free [32P]-αdCTP.

## EXAMPLE 6. ISOLATION OF POLY-A+ RNA

Porcine pituitary total RNA was isolated in substantial accordance with the teaching of Example 1 above. Polyadenylated mRNA was isolated using a Quick Prep™ mRNA Purification Kit (commercially available from Pharmacia, Piscataway, New Jersey) in substantial accordance with Procedures A and B of the instructions provided by the manufacturer. The resulting precipitate RNA was redissolved in an 25 μl volume of sterile water.

For RNA, one $A_{260}$ absorbance unit = 40 μg/ml, the concentration of RNA present in the elute was calculated using the following formula:

$$[RNA] = A_{260} \times D \times 40 \text{ μg/ml}$$

where D=final dilution factor. $A_{260}$ for the elute was found to be 0.4578 with a dilution factor of 100 indicating a RNA concentration of 1831 μg/ml, or approximately 1.8 μg/μl

## EXAMPLE 7. cDNA Synthesis and LAMBDA Cloning

The SuperScript™ Lambda System (commercially available from GIBCO-BRL, Gaithersburg MD) was used for cDNA Synthesis and Cloning of the using isolated poly A+ RNA. 10 μg of Poly A+ RNA was used for cDNA synthesis. The cDNA synthesis was performed in substantial accordance with the protocol described in GIBCO-BRL instructions for the SuperScript™ Lambda System. The packaging of cDNA was performed using the λ Packaging System (commercially available from GIBCO-BRL, Gaithersburg MD as catalog No. 8294SA) in substantial accordance with the instructions provided by the vendor.

## EXAMPLE 8.. Screen cDNA Library

Mix 100 μl packaged cDNA-containing phage to 4 ml of cultured Y1090 (r⁻) cells. Two-hundred μl from infected Y1090 (r⁻) cells was added to 8 ml soft TY agar containing 10 mM MgCl₂ and overlayed on a 150 mm plate with TY agar and incubated overnight at 37°C. Twenty plates (or approximately 1,000,000 phage) were screened. The plates were refrigerated for 2 hours at 4°C before lifting with Hybond-N® nylon membranes to prevent top agar from sticking to the membranes. The phage were transferred for 2 minutes onto Hybond-N® membranes before lifting and marked through the agar for orientation. When making duplicates, the 2nd filter was allowed to transfer for approximately 7 minutes.

The filters were denatured after lifting by submerging in denaturing solution (1.5M NaCl, 0.5M NaOH) for 2 minutes. The filters were neutralized for 5 minutes by submerging in neutralising solution (1.5M NaCl, 0.5M Tris-Cl pH 8.0). The filters were rinsed for approximately 30 seconds only, and then submerged in (0.2M Tris-Cl pH 7.5, 2xSSC). The filters were allowed to air dry on Whatman 3MM paper and baked at 80° under vacuum for two hours.

## EXAMPLE 9. Filter Hybridization

Filter hybridization was performed in substantial accordance with the teaching of Hamilton, et al. Nucleic Acids Research 19:1951-1952 (1991). Briefly, 40 filters are placed in a Seal-a-Meal® bag to which is added 100 ml of the hybridization buffer (50% formamide, 5xSSPE, 1x Denhardts solution, 1% SDS, 100 mg/ml salmon sperm DNA) followed by prehybridization at 42°C for 1 hour. Add 100 μl of the PCR-generated ³²P-probe as described in Example 5 above (total radioactivity 1x10⁹ cpm) to prehybridized filters then hybridize at 42°C overnight. Wash filters 3 times at ambient temperature with 0.1 x SSPE/0.3% SDS with shaking 20 minutes/wash. Repeat wash 3 times at 47°C with 0.1 x SSPE/0.3% SDS with shaking 20 minutes/wash. Mount filter on plastic film and autoradiograph with intensifying screen at -80°C overnight.

### EXAMPLE 10. Making a Liquid Lysate

Host bacteria (Y1090(r-)) were grown to stationary phase overnight and infected with $10^5$ to $10^8$ phage/ml. Following phage adsorption, the infected E. coli culture was diluted into a rich medium and shaken vigorously until cell lysis. Any remaining viable cells were lysed with chloroform. Cell debris was removed by low-speed centrifugation. An overnight culture of a lambda-sensitive strain of E. coli was grown in LB medium at 37°C. Lambda-sensitive strains will support lytic growth. This can be tested by spotting 10 μl of lambda lysate onto a lawn of bacteria. If the strain is lambda-sensitive, a plaque will form where the phage were spotted. Using a sterile toothpick or capillary tube, a single plaque was picked and blown into a tube that contains 0.4 ml lambda dilution buffer and placed at 4°C for 2 hr to allow phage to elute. Alternatively, $10^5$ to $10^8$ phage from a liquid lysate or plate stock can be used.

One-hundred μl eluted phage was combined with 100 μl of saturated culture and 100 μl of 10 mM $MgCl_2$/10 mM $CaCl_2$ solution and incubated 15 min in a 37°C water bath. Incubation with $Mg^{++}$ and $Ca^{++}$ allowed the phage to adsorb to the bacteria. This solution was transferred to 50 ml of NZC medium and shaken vigorously at 37°C until lysis occured (apoproximately 6 and 8 hr). Good aeration is important for high yields. The culture should be checked frequently after 6 hr, and harvested immediately upon clearing.

Add a few drops of chloroform to lyse any remaining cells, the solution was carefully transferred to Corex® or Nalgene® centrifuge tubes (being careful to leave the chloroform behind), and spun 10 min at 10,000 rpm in a Beckman JA-20 rotor to pellet the cell debris. Save as much of the lysate as desired. The solution was transferred to a screw-cap tube, to which a few drops of chloroform were added and the solution briefly vortexed and stored at 4°C. The titer of the phage should be determined as described in Current Protocols in Molecular Biology, and Supplements, Ausubel, et al Eds., (1989 and supplements) John Wiley and Sons, NY. Unit 1.11.

### EXAMPLE 11. ISOLATE PHAGE DNA AND CLONE cDNA INTO PLASMID pSPORT1

The following protocol was used for making small quantities of DNA to be used for restriction analysis. Phage were concentrated by centrifugation and their capsids were destroyed with phenol. The DNA was then ethanol precipitated.

The following additional materials were used in this and following examples:

5 mg/ml DNase (as described in Current Protocols in Molecular Biology, and Supplements, Ausubel, et al Eds.,(1989 and supplements) John Wiley and Sons, NY.Unit 3.12)

10 mg/ml DNase-free RNase (as described in Current Protocols in Molecular Biology, supra Unit 3.13)

0.05 M Tris-Cl, pH 8.0

3 M sodium acetate, to pH 4.8 with acetic acid

To approximately 50 ml liquid phage lysate (Current Protocols in Molecular Biology, supra Unit 1.12), 10 ml of 5 mg/ml DNase and 25 ml of 10 mg/ml DNase-free RNase were added and the reaction mixture was incubated for 1 hr at 37°C to degrade the bacterial DNA and RNA released during lysis. The viscosity of the mixture should decrease. The reaction mixture was centrifuged for 1 1/2 hr at 27,000 rpm in an SW-28 rotor (132,000 xg), 4°C. Alternatively, the phage may be pelleted by spinning 2 1/4 hr in JA-20 rotor at 20,000 rpm (48,000 x g), 4°C.

Resuspend the phage pellet in 200 μl of 0.05 M Tris-Cl, pH 8.0. A small translucent pellet was visible after the tubes are inverted. Transfer the solution to a microcentrifuge tube and add 200 μl buffered phenol and vortex for 20 min or shake for 20 min in microcentrifuge tube shaker. The tube was spun for 2 min in microcentrifuge and the aqueous (top) layer was saved. Phenol extraction was repeated. Phenol denatures the phage capsids and releases the DNA. This denatured capsid protein appeared as a thick white precipitate at the phenol/water interface. Vigorous agitation was necessary to resuspend the pellet. There should be less white precipitate after the second phenol extraction. If there is still a large amount at the interface, do a third extraction.

200 μl chloroform was added, the mixture shaken well, and spun briefly in a microcentrifuge. The aqueous (top) layer was saved and the process repeated. 20 μl of 3 M sodium acetate, pH 4.8, was added and the DNA precipitated with 2 vol of 100% ethanol at room temperature. The mixture was spun in microcentrifuge for 10 min and the supernatant discarded. The pellet was washed with 1 ml of 70% ethanol. The pellet was dried under a vacuum and the DNA resuspended in 25 μl TE buffer, pH 8.0.

The phage DNA was resuspended in 25 μl TE buffer, 5 μl 10x high salt buffer (Boehringer-Mannheim), 16 μl $H_2O$, NotI 2 μl (10 U/μl Boehringer-Mannheim) and SalI 2 μl (10 U/μl Boehringer-Mannheim). The digestion mixture was incubated at 37°C for 2 hours. 50 μl of the digestion mixture was run on a 1% low melting agarose gel. A 1.6 kb cDNA fragment was excised and melted at 65°C. This cDNA fragment was then ligated to a linearized plasmid pSPORT1 in accordance with the following procedure.

Plasmid pSPORT 1 is a multifunctional expression vector for oriented cDNA cloning, in vitro transcription, and dideoxy sequencing. This vector is commercially available from Gibco BRL, Gaithersburg MD. To the 20 μl (1 μg) of NotI-SalI linearized pSPORT 1 was added 30 μl of the 1.6 kb cDNA fragment, 6 μl 10x kinase buffer, 3 μl 10mM ATP pH 7.0 & 2 μl T4 DNA Ligase (1μ/μl BRL). The mixture was incubated overnight at 14°C.

The ligation mixture was melted for 10 minutes at 65°C. 50 μl of melted ligation mixture was added to 200 μ competent DH5α cells on ice and incubated on ice for 30 minutes. One ml of TY broth was added and incubated at 37°C with shaking for 1 hour. The transformed cells in TY broth were plated on TY-Ap(100 μg/ml Ap) agar and incubated overnight at 37°C.

## EXAMPLE 12. Large Scale Plasmid Preparation

Three colonies from the plates prepared in Example 11 were picked and used to prepare large quantities of the plasmid pSPORT1 containing the cDNA fragment. The large scale plasmid was prepared in substantial accordance with the teaching of Sambrook, J., Fritsch, E.F., and Maniatis, T., 1989. Molecular Cloning: A Laboratory Manual, Cold Spring Harbor (1989). Sequence analysis of the cDNA fragment cloned into the plasmid pSPORT1 showed that the cDNA encded a purtative GRF receptor of 451 amino acids. This plasmid was designated as pSPORT-G5

## EXAMPLE 13. CLONING OF G5 cDNA FRAGMENT INTO pRC/CMV VECTOR

### EXAMPLE 13.a. Digestion of plasmid PRC/CMV by Hind III and Not I

22 μl (26 micrograms) of plasmid PRC/CMV (Invitrogen) were digested by 5 μl of Hind III (10U/μl) in 3 μl of 10x M buffer (Boehringer Mannheim) at 37°C for 1.5 hours. Then add 1.3 μl of 1 M Tris-HCl (pH 7.5), 1 μl of 1.5 M NaCl, 0.5 μl of 10x M buffer and 5 μl of Not I (10U/microliter). Incubate the mixture at 37°C for 1.5 hours. Run the 0.7% agarose gel electrophoresis with the reaction mixture and one band (5.4 Kb) was observed. Cut the 5.4 Kb band and purify the vector DNA using Spin-Bind DNA Extraction Units (FMC)

### EXAMPLE 13.b. Preparation of G5 cDNA fragments

30 μg of the starting plasmid, pSPORT-G5 (for GRF receptor cDNA) was digested by 50 units of SalI enzyme in a 50 μl SalI buffer at 37 degrees for 2 hours. The enzymes were inactivated by heating to 70°C for 10 min Twenty μ of 10x nick translation buffer, 16 μl of 2.5 mM dNTP, 104 μl sterile water, 10 μl of Klenow enzyme (2U/microliter, Boeringer-Mannheim) were added and the mixture incubated at room temperature for 30 min. The reaction mixture was heated to 70°C for 10 min and extracted with phenol:chloroform (1:1). The DNA was precipitated with ethanol and resuspended in 35 μl sterile water.

To the 35 μl of the DNA solution, 5 μl of 10x Kinase buffer, 2.5 μl of 10 mM ATP, 5 μl of HindIII linker (1 micrograms/microliter, New England Biolabs) and 2 μl of T4 DNA ligase (1U/μl, Gibco-BRL) were added and incubated at 16°C overnight. The reaction mixture was again extracted with phenol:chloroform (1:1) and the DNA precipatated with ethanol. The DNA pellet was redissolved in 60 μl sterile water. 60 μl of above DNA solution was added to 10 μl of 10x M buffer and 30 μl of HindIII (10 U/μl, Boehringer-Mannheim) and the mixture incubated at 37°C for 2 hours. Four μl of 1 M Tris HCl (pH 7.5), 1.6 μl of 3 M NaCl, 1 microliter of 10x H buffer and 5 μl of NotI (10 U/μl, Boehringer-Mannheim) was then added and reaction mixture was incubated for another two hours at 37°C. The reaction mixture was then heated to 70°C for 10 minutes to inactivate enzymes. The DNA was then electrophoresed on a 0.7% agarose gel. A 1.6 kb cDNA band was excised from the gel and purified by using Spin Bind DNA Extraction Units (FMC Corporation)

### EXAMPLE 13.c. Ligation and transformation

The NotI-HindIII linearized pRC/CMV vector DNA (Example 13.a.) and the 1.6 kb cDNA fragment (Example 13.b.) were ligated in the presence 1x Kinase buffer, 0.5 mM ATP and 1U T4 DNA ligase (Gibco-BRL) at 16°C overnight. The ligation mixture was then transformed into DH5α competent cells (BRL).

### EXAMPLE 13.d. Screen transformants using PCR

Nine transformants from TY/Ap plates (100 μg/ml Ap) were picked and transferred to 2 ml TY/Ap medium and incubated at 37°C for 3 hours. Two μl of each cell cultures was used as a template. Oligonucleotide No.

6110 having the DNA sequence:

```
5'-CCCACTGCTTAACTGGCTTA-3'    (Sequence ID#5)
```

was used as universal upstream primer, oligonucleotide No. 5601 having the DNA sequence:

```
5'-AGAAGGGTTGACTTGGAGAG-3'    (Sequence ID#6)
```

as the internal downstream primer for G5.

Check the PCR products by running 6% PAGE. The band of 1.2 Kb indicates the positive candidate of PRC/CMV-G5. Six out of nine transformants were positive candidates of pRC/CMV-G5.

## EXAMPLE 14. CHARACTERIZATION OF pRC/CMV-G5

### EXAMPLE 14.a. Digestion

pRC/CMV-G5 contains a *Bst* XI unique site in G5 insert fragment. Two of the PRC/CMV-G5 positive candidates were digested with Bst XI (Biolabs) at 55°C for 2 hours and a single band of 7 Kb was observed on the agarose gel. These two PRC/CMV-G5 candidates were then subjected to digestion using HindIII/NotI enzymes (Boehringer-Mannheim). Both of the PRC/CMV-G5 candidates produced the 5.3 Kb fragment of vector DNA and the 1.6 Kb fragment of insert DNA.

### EXAMPLE 14.b. Sequencing the boundary regions between insert and vector

The universal primer of pRC/CMV vector plasmid was used to sequence the boundary region between the insert and the vector (USB sequenase version 2.0 DNA sequencing kit) of two pRC/CMV-G5 candidates. Sequencing data indicated that these two pRC/CMV-G5 have correct pGRF receptor sequences.

## EXAMPLE 15. TRANSIENT EXPRESSION OF G5 IN COSI CELLS

Three plasmids, pRC/CMV-G5 and pRC/CMV were used for transfection to COSI cells.

Grow COSI (SV-40 transformed African Green Monkey kidney) cells in 20 ml EF10 medium (DMEM [Dulbecco modified Eagle's essential medium. BRL] containing 10% Fetal Calf Serum and 1x penicillin-streptomycin) in T75 flask at 37°C and 5% $CO_2$ until the cells cover 40% of the surface of the flask. Trypsinize 5 flasks of cells. Combine them and replate these cells into 12 sterile dishes (10 cm) at $1-2 \times 10^6$ cells/dish. Each dish contains 10 ml of EF10 medium. Incubate cells at 37°C and 5% $CO_2$ overnight.

Mix the 25 µg plasmid DNA (pRC/CMV, pRC/CMV-G5 in 450 microliter of sterile water with 500 microliter of 2x HBS buffer (280 mM NaCl, 10 mM KCl, 1.5 mM $Na_2$ $HPO_4 \cdot 2H_2 0$, 12 mM dextrose, 50 mM HEPES, pH 7.05) in a 5 ml transparent tube (Falcon 2054). Then add 50 µl of 2.5 M $CaCl_2$ dropwise to DNA solutions. Tap the tube a few times. After incubation of the mixture for 20 min at room temperature the mixture becomes turbid (milky). While DNA is being precipitated, wash cells with 7 ml of PBS containing 2x penicillicstreptomycin. Refeed cells with 5 ml of EF10. Add the DNA solution to cells slowly (dropwise) and swirl the plate gently to dispense DNA. Incubate cells at 37°C and 5% $CO_2$ for 5 hours. Remove medium, wash cells once with 10 ml PBS containing 2x penicillin-streptomycin solution. Add 1 ml of 20% DMSO/PBS solution. Keep the cells at room temperature for 45-60 seconds and then remove DMSO/PBS solution as quickly as possible. Wash cells immediately with 10 ml PBS/containing 2x penicillic-streptomycin. Refeed the cells with 12 ml EF 10 medium. Incubate cells at 37°C and 5% $CO_2$ for 48 hours.

## EXAMPLE 16. ISOLATION OF TOTAL RNA FROM TRANSFECTED COSI CELLS.

Total RNA from transfected COS1 cells was isolated using the RNA Isolation Kit from Promega in substantial accordance with the Promega Technical Bulletin included therewith. Use three plates of cells to isolate total RNA. Remove cell culture media. Wash cells with ice chilled sterile PBS buffer. Use 8 ml of prechilled denaturing solution (25 grams of guanadinium thiocynate dissolved in 33ml of CBS [citrate/sarcosine/βmercaptoethanol] buffer) to lyse 3 plates of cells. Transfer the cell lysate into a 50 ml plastic centrifuge tube. To the cell culture was added 0.8 ml of 2 M NaOAc (pH 4.0), mixed well by inversion. Eight ml of phenol:chloroform:isomyl alcohol, was then added and the suspension mixed by inversion, vortexed for 10 seconds and then chilled on ice for fifteen minutes. The suspension was centrifuged (7000 rpm) at 4°C for 20 min. The top aqueous phase which containing the RNA was transferred into a new 50 ml tube. An equal volume of isopropanol was added to the RNA solution. The RNA was pelleted after it precipiated at -20°C overnight. The pellet was washed with ice cold 75% ethanol, dried in vacuo and dissolved in 100 $\mu$l of sterile water.

## Example 17. Northern Blot Analysis

Prepare a 1.1% agarose gel which contains 17% formaldehyde and 1 x MOPS. The RNA sample contains 25 $\mu$g total RNA, 50% formadine, 17% formaldehyde, 1x MOPS, 1x dye and 1 $\mu$g Ethidium bromide (EtBr). Heat the sample at 65°C for 15 min. Cool on ice for 2 min. Run RNA gel in 1x MOPS buffer at 100 voltage for 3 hrs. After taking a photo of the gel under UV, rinse the gel with distilled water once. Soak the gel in 20 x SSC at room temperature for 10 min twice. Transfer the denatured RNA to nylon membrane (Hybond-N) overnight. After blotting, mark the position of the gel slots on the filter. Soak the filter in 6 x SSC at room temperature for 5 min. The RNA was fixed on nylon membrane using a Stratalinker® UV Crosslinker (commercially available from Stratagene, 11099 North Torrey Pines Road, LaJolla CA 92037), the filter was air dried and placed in a plastic bag to which was added a 15 ml of hybridization buffer [1mM EDTA, 0.5M $NaH_2PO_4$, pH 7.2, 7% SDS] and prehybridized at 65°C for 2 hrs with shaking. The solution was removed and another 15 ml of hybridization buffer and [32P]-DNA probe (PCR) at 1 - 2 x $10^8$ cpm, hybridized at 65°C with shaking overnight. The filter was washed in 200 ml of solution containing 1 mM EDTA, 40 mM $NaH_2 PO_4$ (pH 7.2), 1% SDS at 65°C for 1 hour twice. Expose the filter to x-ray film (Kodak XAR 5) at room temperature or -70°C with an intensifying screen for an adequate time.

## EXAMPLE 18. TRANSIENT EXPRESSION OF PRC/CMV-G5 IN 293 CELLS.

Trypsinize exponentially growing 293 cells, inoculate 10 cm plate with 293 cells at 1 x 106 cells/plate and incubate plates at 37°C / 5% $CO_2$ for 2 hrs in 10 ml EF 10 medium per plate.

Plasmids pRC/CMV-G5 and pRC/CMV are used in transfection. Dilute 20 $\mu$g of DNA with sterile Milli-Q water to 450 $\mu$l. Add 50 $\mu$l of 2.5 M $CaCl_2$, 500 $\mu$l of 2 x BBS buffer [N, N-bis (2-hydroxyethyl)2-amino-ethanesulfonic acid and buffered saline, pH 6.95), and mix gently. Incubate the mixture at room temperature for 20 min. Gently mix the milky precipitate, and slowly add 1 ml of the precipitate into the culture, swirl the plate gently to distribute the DNA precipitate evenly. Incubate the plates at 37°C/5% $CO_2$ for 42 hrs. Trypsinize cells of two plates and combine these cells together. Split these cells into tw 6-well plates. Each well contains about $10^6$ cells. Incubate cell culture at 37°C/5% $CO_2$ for 24 hrs.

## EXAMPLE 19. pGRF RECEPTOR EXPRESSION IN STABLY TRANSFECTED 293 CELLS

A full-length GHRH receptor cDNA (G5) containing SalI and NotI ends was modified at the 5'-end (SalI end) by the addition of HindIII linker (Example 13.b.). The cDNA was then ligated to the pRC/CMV vector at the unique HindIII and NotI sites to generate a receptor expression vector, pRC/CMV-G5 (Example 13.c.). The vector pRC/CMV-G5 was then transfected into 293 cells by calcium phosphate co-precipitation procedure (Example 18). Cells were allowed to grow for 3-4 days before adding Geneticin (G-418) to the media at a concentration of 300 $\mu$g/ml for the selection of permanently transfected clones. After 2-3 weeks, 48 clones were selected, propagated and assayed by the cytoplasmic dot hybridization procedure (White and Bancroft 1982) for receptor expression. Ten clones that expressed the receptor were selected for analysis of intracellular cAMP accumulation. The clone (293/G5-12) that gave the highest accumulation of cAMP in response to GHRH challenge was established and used to study the function and binding characteristics of the cloned receptor.

## EXAMPLE 20. cAMP ASSAY

The intracellular cAMP levels were assayed following essentially the procedure decribed by Ishihara et al., (1991) using a cyclic AMP assay kit (Cat. no. TRK.432), commercially available from Amersham Corporation 2636 South Clearbrook Drive, Arlington Heights, IL 60005. In brief, the stable 293 cell line containing the pRC/CMV-G5 was grown to confluency in 24-well plates. Cells were washed twice with the incubation buffer (Dulbecco's modified Eagle's medium containing 0.5 mM l-methyl-3-isobutylxanthine and 1 mg/ml BSA). The cells were then incubated at 37°C for 45 min in the buffer (0.5 ml) containing various concentrations of peptides ($10^{-5}$ to $10^{-10}$ M). After removing the buffer, 0.5 ml of 60% ethanol was added to lyse the cells in each well. An aliquot ( 10 $\mu$l) was taken to be dried in vacuo and the level of cAMP was determined using the Cyclic AMP Assay Kit (Amersham, catalog No. TRK 432).

## EXAMPLE 21. BINDING OF $^{125}$I-GRF TO 293/G5-12 CELLS

Binding assays were performed on a monolayer 293/G5-12 culture grown in 48-well plates. The wells were precoated with poly-D-lysine in order to increase cell attachment to the surface. The 293/G5-12 cells expressing the GRF receptor were allowed to grow to confluency in the culture medium EF10 containing 300 µg/ml Genticin (G418). The confluent cells (approximately $10^5$ cells/well) were then washed twice with cold receptor assay buffer (Hepes-buffered saline containing 0.1% protease-free BSA). The radioreceptor assay was then performed in triplicate by using 0.125 nM of $^{125}$I-GRF in the incubation buffer and each of three unlabeled hormone competitors (porcine GRF, VIP and secretin) at various concentrations ($10^{-5}$ $10^{-10}$ M). The incubation was performed for 45-60 minutes at 4°C and the cells washed three times with Hank's Balanced Salt solution. [125]]-GRF bound to the cells was then determined in a gamma counter after the cells were lysed with 0.5 ml of 0.5 N NaOH in each well.

## EXAMPLE 22. Isolation of G3 pGRF Receptor Isoform

The pGRF designated G3, the amino acid and DNA sequence of which are designated Sequence ID #12 and Sequence ID#11 respectively, was isolated in substantial accordance with the teaching of Examples 1 through 19 herein. The determination that the G3 receptor is expressed in stably transformed 293 cells (designated 293/G3-12) was performed in substantial accordance with the teach of Example 19 herein. The response of the G3 receptor to activate adenylate cyclase as reported in Table 5 above was measured using the assay procedure of Example 20 herein.

SEQUENCE LISTING

(1) GENERAL INFORMATION:

    (i) APPLICANT: ELI LILLY AND COMPANY
        (B)   STREET:  Lilly Corporate Center
        (C)   CITY:  Indianapolis
        (D)   STATE:  Indiana
        (E)   COUNTRY:  United States of America
        (F)   ZIP: 46285

    (ii) TITLE OF INVENTION: PORCINE GRF RECEPTOR

    (iii) NUMBER OF SEQUENCES: 12

    (iv) CORRESPONDENCE ADDRESS:
        (A) ADDRESSEE: C. M. Hudson
        (B) STREET: Erl Wood Manor
        (C) CITY: Windlesham
        (D) STATE: Surrey
        (E) COUNTRY: United Kingdom
        (F) ZIP: GU20 6PH

    (v) COMPUTER READABLE FORM:
        (A) MEDIUM TYPE: Floppy disk
        (B) COMPUTER: Macintosh
        (C) OPERATING SYSTEM: System 7.0
        (D) SOFTWARE: Microsoft Word version 5.1

(2) INFORMATION FOR SEQ ID NO: 1:

    (i) SEQUENCE CHARACTERISTICS:
        (A) LENGTH: 1356 base pairs
        (B) TYPE: nucleic acid
        (C) STRANDEDNESS: single
        (D) TOPOLOGY: linear

    (ii) MOLECULE TYPE: DNA (genomic)

    (ix) FEATURE:
        (A) NAME/KEY: CDS
        (B) LOCATION: 1..1356

    (xi) SEQUENCE DESCRIPTION: SEQ ID NO: 1:

```
ATG GAC AGC GGG GTG TGG GCT GCC TGC ATC TTC TGC CTG CTG AGC TCC       48
Met Asp Ser Gly Val Trp Ala Ala Cys Ile Phe Cys Leu Leu Ser Ser
 1               5                  10                  15

CTA CCA GTC GCC TTG GGC CAC GTG CAC CCG GAA TGT GAC TTC ATC ACC       96
Leu Pro Val Ala Leu Gly His Val His Pro Glu Cys Asp Phe Ile Thr
                20                  25                  30

CAG CTG AGA GAA GAC GAG CGA ACT TGT CTA CAA GCA GCA GAC CGG ATG      144
Gln Leu Arg Glu Asp Glu Arg Thr Cys Leu Gln Ala Ala Asp Arg Met
            35                  40                  45
```

```
GCC AAC TCC TCC TCG GGC TGT CCT AGG ACC TGG GAT GGG CTG TTG TGC        192
Ala Asn Ser Ser Ser Gly Cys Pro Arg Thr Trp Asp Gly Leu Leu Cys
     50              55                  60

TGG CCG ACG GCA GGC CCT GGG GAG TGG GTG ACC CTC CCC TGC CCG GCT        240
Trp Pro Thr Ala Gly Pro Gly Glu Trp Val Thr Leu Pro Cys Pro Ala
65              70                  75                  80

TTC TTC TCT CAC TTC AGC TCT GAG CCA GGG GCC CTG AAG CGG GAC TGC        288
Phe Phe Ser His Phe Ser Ser Glu Pro Gly Ala Leu Lys Arg Asp Cys
                85                  90                  95

ACC ACC ACG GGC TGG TCT GAG CCC TTC CCG CCA TAT CCC GAG GCT TGC        336
Thr Thr Thr Gly Trp Ser Glu Pro Phe Pro Pro Tyr Pro Glu Ala Cys
            100                 105                 110

CCT GTG CCC CTG GAG CTG CTG ACT GAT GAG AAA TCC TAC TTC TCC ACT        384
Pro Val Pro Leu Glu Leu Leu Thr Asp Glu Lys Ser Tyr Phe Ser Thr
        115                 120                 125

GTG AGA ATC GTC TAC ACC ACG GGC CAC AGC GTC TCT GCC GTG GCC CTC        432
Val Arg Ile Val Tyr Thr Thr Gly His Ser Val Ser Ala Val Ala Leu
    130                 135                 140

TTC GTG GCC ATC GCC ATC CTG GTT GCT CTC AGG AGG CTC CAC TGC CCC        480
Phe Val Ala Ile Ala Ile Leu Val Ala Leu Arg Arg Leu His Cys Pro
145                 150                 155                 160

AGG AAC TAC ATC CAC AGC CAG CTG TTC GCC ACC TTT ATC CTC AAG GCG        528
Arg Asn Tyr Ile His Ser Gln Leu Phe Ala Thr Phe Ile Leu Lys Ala
                165                 170                 175

GGA GCT GTG TTC TTG AAA GAC GCC GCC CTC TTT CAC AGC GAG AAC ACG        576
Gly Ala Val Phe Leu Lys Asp Ala Ala Leu Phe His Ser Glu Asn Thr
            180                 185                 190

GAC CAC TGC AGC TTC TCC ACG GTT CTG TGC AAG GTC TCT GTG GCC ACC        624
Asp His Cys Ser Phe Ser Thr Val Leu Cys Lys Val Ser Val Ala Thr
            195                 200                 205

TCC CAT TTC GCC ACC ATG ACC AAC TTC AGC TGG CTG CTG GCA GAA GCT        672
Ser His Phe Ala Thr Met Thr Asn Phe Ser Trp Leu Leu Ala Glu Ala
        210                 215                 220

GTC TAC CTG ACC TGC CTC TTG GCC TCT ACG TCA CCC AGC ACG AGG AGG        720
Val Tyr Leu Thr Cys Leu Leu Ala Ser Thr Ser Pro Ser Thr Arg Arg
225                 230                 235                 240

GCC TTC TGG TGG CTG GTT CTC GCT GGC TGG GGG CTG CCC CTG CTC TTC        768
Ala Phe Trp Trp Leu Val Leu Ala Gly Trp Gly Leu Pro Leu Leu Phe
                245                 250                 255

ACT GGC ACG TGG GTG GGT TGC AAG TTG GCC TTT GAG GAT GTT GCG TGC        816
Thr Gly Thr Trp Val Gly Cys Lys Leu Ala Phe Glu Asp Val Ala Cys
            260                 265                 270
```

```
TGG GAC CTG GAC GAC AGC TCC CCC TAC TGG TGG ATC ATC AAA GGG CCC      864
Trp Asp Leu Asp Asp Ser Ser Pro Tyr Trp Trp Ile Ile Lys Gly Pro
        275                 280                 285

ATC GTC CTC TCC GTG GGG GTG AAC TTT GGG CTT TTT CTC AAT ATT ATC      912
Ile Val Leu Ser Val Gly Val Asn Phe Gly Leu Phe Leu Asn Ile Ile
        290                 295                 300

CGC ATC CTG CTG AGG AAA CTG GAG CCA GCT CAG GGC AGC CTC CAC ACC      960
Arg Ile Leu Leu Arg Lys Leu Glu Pro Ala Gln Gly Ser Leu His Thr
305                 310                 315                 320

CAG CCT CAG TAC TGG CGT CTC TCC AAG TCA ACC CTT CTC CTC ATC CCG     1008
Gln Pro Gln Tyr Trp Arg Leu Ser Lys Ser Thr Leu Leu Leu Ile Pro
                325                 330                 335

CTG TTT GGA ATT CAC TAC GTC ATC TTC AAC TTC CTG CCT GAC AGT GCT     1056
Leu Phe Gly Ile His Tyr Val Ile Phe Asn Phe Leu Pro Asp Ser Ala
            340                 345                 350

GGC CTG GGC ATC CGC CTC CCC CTG GAG CTG GGA CTG GGC TCC TTC CAG     1104
Gly Leu Gly Ile Arg Leu Pro Leu Glu Leu Gly Leu Gly Ser Phe Gln
            355                 360                 365

GGC TTC ATT GTT GCC ATC CTG TAC TGC TTC CTC AAC CAA GAG GTG AGG     1152
Gly Phe Ile Val Ala Ile Leu Tyr Cys Phe Leu Asn Gln Glu Val Arg
        370                 375                 380

ACT GAG ATC TCA CGG AGG TGG CAT GGC CAT GAC CCT GAA CTT CTG CCA     1200
Thr Glu Ile Ser Arg Arg Trp His Gly His Asp Pro Glu Leu Leu Pro
385                 390                 395                 400

GCC TGG AGG ACT CAT GCC AAA TGG GCA AAG CCT TCC CGC TCA AGG GCG     1248
Ala Trp Arg Thr His Ala Lys Trp Ala Lys Pro Ser Arg Ser Arg Ala
                405                 410                 415

AAG GTC AGT GCG TGT TCA CGT GCT GGG TCT TCC CGC CCC CGA GCC CAT     1296
Lys Val Ser Ala Cys Ser Arg Ala Gly Ser Ser Arg Pro Arg Ala His
                420                 425                 430

GGC GAC ACA TAC CCG GGA CTG GAA GTG CCG GGT CAG TGG TTG TGC CTT     1344
Gly Asp Thr Tyr Pro Gly Leu Glu Val Pro Gly Gln Trp Leu Cys Leu
                435                 440                 445

TTC TTG ACG TAG                                                     1356
Phe Leu Thr
        450
```

(2) INFORMATION FOR SEQ ID NO: 2:

      (i) SEQUENCE CHARACTERISTICS:
          (A) LENGTH: 451 amino acids
          (B) TYPE: amino acid
          (D) TOPOLOGY: linear

      (ii) MOLECULE TYPE: protein

(xi) SEQUENCE DESCRIPTION: SEQ ID NO: 2:

```
Met Asp Ser Gly Val Trp Ala Ala Cys Ile Phe Cys Leu Leu Ser Ser
 1               5                  10                      15

Leu Pro Val Ala Leu Gly His Val His Pro Glu Cys Asp Phe Ile Thr
            20                  25                  30

Gln Leu Arg Glu Asp Glu Arg Thr Cys Leu Gln Ala Ala Asp Arg Met
        35                  40                  45

Ala Asn Ser Ser Ser Gly Cys Pro Arg Thr Trp Asp Gly Leu Leu Cys
    50                  55                  60

Trp Pro Thr Ala Gly Pro Gly Glu Trp Val Thr Leu Pro Cys Pro Ala
65                  70                  75                      80

Phe Phe Ser His Phe Ser Ser Glu Pro Gly Ala Leu Lys Arg Asp Cys
                85                  90                  95

Thr Thr Thr Gly Trp Ser Glu Pro Phe Pro Pro Tyr Pro Glu Ala Cys
            100                 105                 110

Pro Val Pro Leu Glu Leu Leu Thr Asp Glu Lys Ser Tyr Phe Ser Thr
            115                 120                 125

Val Arg Ile Val Tyr Thr Thr Gly His Ser Val Ser Ala Val Ala Leu
        130                 135                 140

Phe Val Ala Ile Ala Ile Leu Val Ala Leu Arg Arg Leu His Cys Pro
145                 150                 155                     160

Arg Asn Tyr Ile His Ser Gln Leu Phe Ala Thr Phe Ile Leu Lys Ala
                165                 170                 175

Gly Ala Val Phe Leu Lys Asp Ala Ala Leu Phe His Ser Glu Asn Thr
            180                 185                 190

Asp His Cys Ser Phe Ser Thr Val Leu Cys Lys Val Ser Val Ala Thr
        195                 200                 205

Ser His Phe Ala Thr Met Thr Asn Phe Ser Trp Leu Leu Ala Glu Ala
    210                 215                 220

Val Tyr Leu Thr Cys Leu Leu Ala Ser Thr Ser Pro Ser Thr Arg Arg
225                 230                 235                     240

Ala Phe Trp Trp Leu Val Leu Ala Gly Trp Gly Leu Pro Leu Leu Phe
                245                 250                 255

Thr Gly Thr Trp Val Gly Cys Lys Leu Ala Phe Glu Asp Val Ala Cys
            260                 265                 270

Trp Asp Leu Asp Asp Ser Ser Pro Tyr Trp Trp Ile Ile Lys Gly Pro
            275                 280                 285
```

```
Ile Val Leu Ser Val Gly Val Asn Phe Gly Leu Phe Leu Asn Ile Ile
    290             295             300

Arg Ile Leu Leu Arg Lys Leu Glu Pro Ala Gln Gly Ser Leu His Thr
305             310             315                 320

Gln Pro Gln Tyr Trp Arg Leu Ser Lys Ser Thr Leu Leu Leu Ile Pro
            325             330                 335

Leu Phe Gly Ile His Tyr Val Ile Phe Asn Phe Leu Pro Asp Ser Ala
            340             345             350

Gly Leu Gly Ile Arg Leu Pro Leu Glu Leu Gly Leu Gly Ser Phe Gln
        355             360             365

Gly Phe Ile Val Ala Ile Leu Tyr Cys Phe Leu Asn Gln Glu Val Arg
    370             375             380

Thr Glu Ile Ser Arg Arg Trp His Gly His Asp Pro Glu Leu Leu Pro
385             390             395                 400

Ala Trp Arg Thr His Ala Lys Trp Ala Lys Pro Ser Arg Ser Arg Ala
            405             410             415

Lys Val Ser Ala Cys Ser Arg Ala Gly Ser Ser Arg Pro Arg Ala His
            420             425             430

Gly Asp Thr Tyr Pro Gly Leu Glu Val Pro Gly Gln Trp Leu Cys Leu
        435             440             445

Phe Leu Thr
    450
```

(2) INFORMATION FOR SEQ ID NO: 3:

    (i) SEQUENCE CHARACTERISTICS:
       (A) LENGTH: 17 base pairs
       (B) TYPE: nucleic acid
       (C) STRANDEDNESS: single
       (D) TOPOLOGY: linear

    (ii) MOLECULE TYPE: DNA (genomic)

    (xi) SEQUENCE DESCRIPTION: SEQ ID NO: 3:

GTAAAACGAC GGCCAGT                        17

(2) INFORMATION FOR SEQ ID NO: 4:

    (i) SEQUENCE CHARACTERISTICS:
       (A) LENGTH: 17 base pairs
       (B) TYPE: nucleic acid
       (C) STRANDEDNESS: single
       (D) TOPOLOGY: linear

    (ii) MOLECULE TYPE: DNA (genomic)

(xi) SEQUENCE DESCRIPTION: SEQ ID NO: 4:

CAGGAAACAG CTATGAC                                                    17

(2) INFORMATION FOR SEQ ID NO: 5:

     (i) SEQUENCE CHARACTERISTICS:
        (A) LENGTH: 20 base pairs
        (B) TYPE: nucleic acid
        (C) STRANDEDNESS: single
        (D) TOPOLOGY: linear

     (ii) MOLECULE TYPE: DNA (genomic)

     (xi) SEQUENCE DESCRIPTION: SEQ ID NO: 5:

CCCACTGCTT AACTGGCTTA                                                 20

(2) INFORMATION FOR SEQ ID NO: 6:

     (i) SEQUENCE CHARACTERISTICS:
        (A) LENGTH: 20 base pairs
        (B) TYPE: nucleic acid
        (C) STRANDEDNESS: single
        (D) TOPOLOGY: linear

     (ii) MOLECULE TYPE: DNA (genomic)

     (xi) SEQUENCE DESCRIPTION: SEQ ID NO: 6:

AGAAGGGTTG ACTTGGAGAG                                                 20

(2) INFORMATION FOR SEQ ID NO: 7:

     (i) SEQUENCE CHARACTERISTICS:
        (A) LENGTH: 1290 base pairs
        (B) TYPE: nucleic acid
        (C) STRANDEDNESS: single
        (D) TOPOLOGY: linear

     (ii) MOLECULE TYPE: DNA (genomic)

     (ix) FEATURE:
        (A) NAME/KEY: CDS
        (B) LOCATION: 1..1290

     (xi) SEQUENCE DESCRIPTION: SEQ ID NO: 7:

```
CAC GTG CAC CCG GAA TGT GAC TTC ATC ACC CAG CTG AGA GAA GAC GAG    48
His Val His Pro Glu Cys Asp Phe Ile Thr Gln Leu Arg Glu Asp Glu
 1               5                   10                  15

CGA ACT TGT CTA CAA GCA GCA GAC CGG ATG GCC AAC TCC TCC TCG GGC    96
Arg Thr Cys Leu Gln Ala Ala Asp Arg Met Ala Asn Ser Ser Ser Gly
            20                  25                  30
```

```
TGT CCT AGG ACC TGG GAT GGG CTG TTG TGC TGG CCG ACG GCA GGC CCT        144
Cys Pro Arg Thr Trp Asp Gly Leu Leu Cys Trp Pro Thr Ala Gly Pro
        35                  40                  45

GGG GAG TGG GTG ACC CTC CCC TGC CCG GCT TTC TTC TCT CAC TTC AGC        192
Gly Glu Trp Val Thr Leu Pro Cys Pro Ala Phe Phe Ser His Phe Ser
    50                  55                  60

TCT GAG CCA GGG GCC CTG AAG CGG GAC TGC ACC ACC ACG GGC TGG TCT        240
Ser Glu Pro Gly Ala Leu Lys Arg Asp Cys Thr Thr Thr Gly Trp Ser
65                  70                  75                  80

GAG CCC TTC CCG CCA TAT CCC GAG GCT TGC CCT GTG CCC CTG GAG CTG        288
Glu Pro Phe Pro Pro Tyr Pro Glu Ala Cys Pro Val Pro Leu Glu Leu
                85                  90                  95

CTG ACT GAT GAG AAA TCC TAC TTC TCC ACT GTG AGA ATC GTC TAC ACC        336
Leu Thr Asp Glu Lys Ser Tyr Phe Ser Thr Val Arg Ile Val Tyr Thr
                100                 105                 110

ACG GGC CAC AGC GTC TCT GCC GTG GCC CTC TTC GTG GCC ATC GCC ATC        384
Thr Gly His Ser Val Ser Ala Val Ala Leu Phe Val Ala Ile Ala Ile
            115                 120                 125

CTG GTT GCT CTC AGG AGG CTC CAC TGC CCC AGG AAC TAC ATC CAC AGC        432
Leu Val Ala Leu Arg Arg Leu His Cys Pro Arg Asn Tyr Ile His Ser
            130                 135                 140

CAG CTG TTC GCC ACC TTT ATC CTC AAG GCG GGA GCT GTG TTC TTG AAA        480
Gln Leu Phe Ala Thr Phe Ile Leu Lys Ala Gly Ala Val Phe Leu Lys
145                 150                 155                 160

GAC GCC GCC CTC TTT CAC AGC GAG AAC ACG GAC CAC TGC AGC TTC TCC        528
Asp Ala Ala Leu Phe His Ser Glu Asn Thr Asp His Cys Ser Phe Ser
                165                 170                 175

ACG GTT CTG TGC AAG GTC TCT GTG GCC ACC TCC CAT TTC GCC ACC ATG        576
Thr Val Leu Cys Lys Val Ser Val Ala Thr Ser His Phe Ala Thr Met
            180                 185                 190

ACC AAC TTC AGC TGG CTG CTG GCA GAA GCT GTC TAC CTG ACC TGC CTC        624
Thr Asn Phe Ser Trp Leu Leu Ala Glu Ala Val Tyr Leu Thr Cys Leu
            195                 200                 205

TTG GCC TCT ACG TCA CCC AGC ACG AGG AGG GCC TTC TGG TGG CTG GTT        672
Leu Ala Ser Thr Ser Pro Ser Thr Arg Arg Ala Phe Trp Trp Leu Val
            210                 215                 220

CTC GCT GGC TGG GGG CTG CCC CTG CTC TTC ACT GGC ACG TGG GTG GGT        720
Leu Ala Gly Trp Gly Leu Pro Leu Leu Phe Thr Gly Thr Trp Val Gly
225                 230                 235                 240

TGC AAG TTG GCC TTT GAG GAT GTT GCG TGC TGG GAC CTG GAC GAC AGC        768
Cys Lys Leu Ala Phe Glu Asp Val Ala Cys Trp Asp Leu Asp Asp Ser
                245                 250                 255
```

```
TCC CCC TAC TGG TGG ATC ATC AAA GGG CCC ATC GTC CTC TCC GTG GGG          816
Ser Pro Tyr Trp Trp Ile Ile Lys Gly Pro Ile Val Leu Ser Val Gly
        260                 265                 270

GTG AAC TTT GGG CTT TTT CTC AAT ATT ATC CGC ATC CTG CTG AGG AAA          864
Val Asn Phe Gly Leu Phe Leu Asn Ile Ile Arg Ile Leu Leu Arg Lys
        275                 280                 285

CTG GAG CCA GCT CAG GGC AGC CTC CAC ACC CAG CCT CAG TAC TGG CGT          912
Leu Glu Pro Ala Gln Gly Ser Leu His Thr Gln Pro Gln Tyr Trp Arg
        290                 295                 300

CTC TCC AAG TCA ACC CTT CTC CTC ATC CCG CTG TTT GGA ATT CAC TAC          960
Leu Ser Lys Ser Thr Leu Leu Leu Ile Pro Leu Phe Gly Ile His Tyr
305                 310                 315                 320

GTC ATC TTC AAC TTC CTG CCT GAC AGT GCT GGC CTG GGC ATC CGC CTC          1008
Val Ile Phe Asn Phe Leu Pro Asp Ser Ala Gly Leu Gly Ile Arg Leu
                325                 330                 335

CCC CTG GAG CTG GGA CTG GGC TCC TTC CAG GGC TTC ATT GTT GCC ATC          1056
Pro Leu Glu Leu Gly Leu Gly Ser Phe Gln Gly Phe Ile Val Ala Ile
                340                 345                 350

CTG TAC TGC TTC CTC AAC CAA GAG GTG AGG ACT GAG ATC TCA CGG AGG          1104
Leu Tyr Cys Phe Leu Asn Gln Glu Val Arg Thr Glu Ile Ser Arg Arg
            355                 360                 365

TGG CAT GGC CAT GAC CCT GAA CTT CTG CCA GCC TGG AGG ACT CAT GCC          1152
Trp His Gly His Asp Pro Glu Leu Leu Pro Ala Trp Arg Thr His Ala
        370                 375                 380

AAA TGG GCA AAG CCT TCC CGC TCA AGG GCG AAG GTC AGT GCG TGT TCA          1200
Lys Trp Ala Lys Pro Ser Arg Ser Arg Ala Lys Val Ser Ala Cys Ser
385                 390                 395                 400

CGT GCT GGG TCT TCC CGC CCC CGA GCC CAT GGC GAC ACA TAC CCG GGA          1248
Arg Ala Gly Ser Ser Arg Pro Arg Ala His Gly Asp Thr Tyr Pro Gly
                405                 410                 415

CTG GAA GTG CCG GGT CAG TGG TTG TGC CTT TTC TTG ACG TAG             1290
Leu Glu Val Pro Gly Gln Trp Leu Cys Leu Phe Leu Thr
        420                 425
```

(2) INFORMATION FOR SEQ ID NO: 8:

    (i) SEQUENCE CHARACTERISTICS:
      (A) LENGTH: 429 amino acids
      (B) TYPE: amino acid
      (D) TOPOLOGY: linear

  (ii) MOLECULE TYPE: protein

  (xi) SEQUENCE DESCRIPTION: SEQ ID NO: 8:

```
His Val His Pro Glu Cys Asp Phe Ile Thr Gln Leu Arg Glu Asp Glu
  1               5               10                  15

Arg Thr Cys Leu Gln Ala Ala Asp Arg Met Ala Asn Ser Ser Ser Gly
              20              25                  30

Cys Pro Arg Thr Trp Asp Gly Leu Leu Cys Trp Pro Thr Ala Gly Pro
          35              40              45

Gly Glu Trp Val Thr Leu Pro Cys Pro Ala Phe Phe Ser His Phe Ser
      50              55              60

Ser Glu Pro Gly Ala Leu Lys Arg Asp Cys Thr Thr Thr Gly Trp Ser
 65              70              75                  80

Glu Pro Phe Pro Pro Tyr Pro Glu Ala Cys Pro Val Pro Leu Glu Leu
              85              90                  95

Leu Thr Asp Glu Lys Ser Tyr Phe Ser Thr Val Arg Ile Val Tyr Thr
          100             105             110

Thr Gly His Ser Val Ser Ala Val Ala Leu Phe Val Ala Ile Ala Ile
          115             120             125

Leu Val Ala Leu Arg Arg Leu His Cys Pro Arg Asn Tyr Ile His Ser
      130             135             140

Gln Leu Phe Ala Thr Phe Ile Leu Lys Ala Gly Ala Val Phe Leu Lys
145             150             155                 160

Asp Ala Ala Leu Phe His Ser Glu Asn Thr Asp His Cys Ser Phe Ser
              165             170             175

Thr Val Leu Cys Lys Val Ser Val Ala Thr Ser His Phe Ala Thr Met
          180             185             190

Thr Asn Phe Ser Trp Leu Leu Ala Glu Ala Val Tyr Leu Thr Cys Leu
          195             200             205

Leu Ala Ser Thr Ser Pro Ser Thr Arg Arg Ala Phe Trp Trp Leu Val
      210             215             220

Leu Ala Gly Trp Gly Leu Pro Leu Leu Phe Thr Gly Thr Trp Val Gly
225             230             235                 240

Cys Lys Leu Ala Phe Glu Asp Val Ala Cys Trp Asp Leu Asp Asp Ser
              245             250             255

Ser Pro Tyr Trp Trp Ile Ile Lys Gly Pro Ile Val Leu Ser Val Gly
          260             265             270

Val Asn Phe Gly Leu Phe Leu Asn Ile Ile Arg Ile Leu Leu Arg Lys
      275             280             285

Leu Glu Pro Ala Gln Gly Ser Leu His Thr Gln Pro Gln Tyr Trp Arg
      290             295             300
```

33

```
Leu Ser Lys Ser Thr Leu Leu Leu Ile Pro Leu Phe Gly Ile His Tyr
305             310             315                         320

Val Ile Phe Asn Phe Leu Pro Asp Ser Ala Gly Leu Gly Ile Arg Leu
            325             330                     335

Pro Leu Glu Leu Gly Leu Gly Ser Phe Gln Gly Phe Ile Val Ala Ile
        340             345                 350

Leu Tyr Cys Phe Leu Asn Gln Glu Val Arg Thr Glu Ile Ser Arg Arg
        355             360             365

Trp His Gly His Asp Pro Glu Leu Leu Pro Ala Trp Arg Thr His Ala
    370             375             380

Lys Trp Ala Lys Pro Ser Arg Ser Arg Ala Lys Val Ser Ala Cys Ser
385             390                 395                     400

Arg Ala Gly Ser Ser Arg Pro Arg Ala His Gly Asp Thr Tyr Pro Gly
            405                 410                 415

Leu Glu Val Pro Gly Gln Trp Leu Cys Leu Phe Leu Thr
            420             425
```

(2) INFORMATION FOR SEQ ID NO: 9:

    (i) SEQUENCE CHARACTERISTICS:
        (A) LENGTH: 27 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO: 9:

CTGCACCTCA CCATTGAGGA AGCAGTA                    27

(2) INFORMATION FOR SEQ ID NO: 10:

    (i) SEQUENCE CHARACTERISTICS:
        (A) LENGTH: 32 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO: 10:

TTCCGGAGGC TGCAYTGCAC YCGMAACTAC AT              32

(2) INFORMATION FOR SEQ ID NO:11:

(i) SEQUENCE CHARACTERISTICS:
(A) LENGTH: 1272 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS (B) LOCATION: 1..1272

(xi) SEQUENCE DESCRIPTION: SEQ ID NO:11:

```
ATG GAC AGC GGG GTG TGG GCT GCC TGC ATC TTC TGC CTG CTG AGC TCC    48
Met Asp Ser Gly Val Trp Ala Ala Cys Ile Phe Cys Leu Leu Ser Ser
1               5                   10                  15

CTA CCA GTC GCC TTG GGC CAC GTG CAC CCG GAA TGT GAC TTC ATC ACC    96
Leu Pro Val Ala Leu Gly His Val His Pro Glu Cys Asp Phe Ile Thr
                20                  25                  30

CAG CTG AGA GAA GAC GAG CGA ACT TGT CTA CAA GCA GCA GAC CGG ATG    144
Gln Leu Arg Glu Asp Glu Arg Thr Cys Leu Gln Ala Ala Asp Arg Met
            35                  40                  45

GCC AAC TCC TCC TCG GGC TGT CCT AGG ACC TGG GAT GGG CTG TTG TGC    192
Ala Asn Ser Ser Ser Gly Cys Pro Arg Thr Trp Asp Gly Leu Leu Cys
        50                  55                  60

TGG CCG ACG GCA GGC CCT GGG GAG TGG GTG ACC CTC CCC TGC CCG GCT    240
Trp Pro Thr Ala Gly Pro Gly Glu Trp Val Thr Leu Pro Cys Pro Ala
65                  70                  75                  80

TTC TTC TCT CAC TTC AGC TCT GAG CCA GGG GCC CTG AAG CGG GAC TGC    288
Phe Phe Ser His Phe Ser Ser Glu Pro Gly Ala Leu Lys Arg Asp Cys
                85                  90                  95

ACC ACC ACG GGC TGG TCT GAG CCC TTC CCG CCA TAT CCC GAG GCT TGC    336
Thr Thr Thr Gly Trp Ser Glu Pro Phe Pro Pro Tyr Pro Glu Ala Cys
                100                 105                 110

CCT GTG CCC CTG GAG CTG CTG ACT GAT GAG AAA TCC TAC TTC TCC ACT    384
Pro Val Pro Leu Glu Leu Leu Thr Asp Glu Lys Ser Tyr Phe Ser Thr
            115                 120                 125

GTG AGA ATC GTC TAC ACC ACG GGC CAC AGC GTC TCT GCC GTG GCC CTC    432
Val Arg Ile Val Tyr Thr Thr Gly His Ser Val Ser Ala Val Ala Leu
        130                 135                 140
```

```
TTC GTG GCC ATC GCC ATC CTG GTT GCT CTC AGG AGG CTC CAC TGC CCC          480
Phe Val Ala Ile Ala Ile Leu Val Ala Leu Arg Arg Leu His Cys Pro
145             150             155             160

AGG AAC TAC ATC CAC AGC CAG CTG TTC GCC ACC TTT ATC CTC AAG GCG          528
Arg Asn Tyr Ile His Ser Gln Leu Phe Ala Thr Phe Ile Leu Lys Ala
            165             170             175

GGA GCT GTG TTC TTG AAA GAC GCC GCC CTC TTT CAC AGC GAG AAC ACG          576
Gly Ala Val Phe Leu Lys Asp Ala Ala Leu Phe His Ser Glu Asn Thr
            180             185             190

GAC CAC TGC AGC TTC TCC ACG GTT CTG TGC AAG GTC TCT GTG GCC ACC          624
Asp His Cys Ser Phe Ser Thr Val Leu Cys Lys Val Ser Val Ala Thr
            195             200             205

TCC CAT TTC GCC ACC ATG ACC AAC TTC AGC TGG CTG CTG GCA GAA GCT          672
Ser His Phe Ala Thr Met Thr Asn Phe Ser Trp Leu Leu Ala Glu Ala
            210             215             220

GTC TAC CTG ACC TGC CTC TTG GCC TCT ACG TCA CCC AGC ACG AGG AGG          720
Val Tyr Leu Thr Cys Leu Leu Ala Ser Thr Ser Pro Ser Thr Arg Arg
225             230             235             240

GCC TTC TGG TGG CTG GTT CTC GCT GGC TGG GGG CTG CCC CTG CTC TTC          768
Ala Phe Trp Trp Leu Val Leu Ala Gly Trp Gly Leu Pro Leu Leu Phe
            245             250             255

ACT GGC ACG TGG GTG GGT TGC AAG TTG GCC TTT GAG GAT GTT GCG TGC          816
Thr Gly Thr Trp Val Gly Cys Lys Leu Ala Phe Glu Asp Val Ala Cys
            260             265             270

TGG GAT CTG GAC GAC AGC TCC CCC TAC TGG TGG ATC ATC AAA GGG CCC          864
Trp Asp Leu Asp Asp Ser Ser Pro Tyr Trp Trp Ile Ile Lys Gly Pro
            275             280             285

ATC GTC CTC TCC GTG GGG GTG AAC TTT GGG CTT TTT CTC AAT ATT ATC          912
Ile Val Leu Ser Val Gly Val Asn Phe Gly Leu Phe Leu Asn Ile Ile
            290             295             300

CGC ATC CTG CTG AGG AAA CTG GAG CCA GCT CAG GGC AGC CTC CAC ACC          960
Arg Ile Leu Leu Arg Lys Leu Glu Pro Ala Gln Gly Ser Leu His Thr
305             310             315             320

CAG CCT CAG TAC TGG CGT CTC TCC AAG TCA ACC CTT CTC CTC ATC CCA          1008
Gln Pro Gln Tyr Trp Arg Leu Ser Lys Ser Thr Leu Leu Leu Ile Pro
            325             330             335

CTG TTT GGA ATC CAC TAC GTC ATC TTC AAC TTC CTG CCT GAC AGT GCT          1056
Leu Phe Gly Ile His Tyr Val Ile Phe Asn Phe Leu Pro Asp Ser Ala
            340             345             350
```

```
GGC CTG GGC ATC CGC CTC CCC CTG GAG CTG GGA CTG GGC TCC TTC CAG      1104
Gly Leu Gly Ile Arg Leu Pro Leu Glu Leu Gly Leu Gly Ser Phe Gln
        355                 360                 365

GGC TTC ATT GTT GCC ATC CTG TAC TGC TTC CTC AAC CAA GAG GTG AGG      1152
Gly Phe Ile Val Ala Ile Leu Tyr Cys Phe Leu Asn Gln Glu Val Arg
    370                 375                 380

ACT GAG ATC TCA CGG AGG TGG CAT GGC CAT GAC CCT GAA CTT CTG CCA      1200
Thr Glu Ile Ser Arg Arg Trp His Gly His Asp Pro Glu Leu Leu Pro
385                 390                 395                 400

GCC TGG AGG ACT CAT GCC AAA TGG GCA AAG CCT TCC CGC TCA AGG GCG      1248
Ala Trp Arg Thr His Ala Lys Trp Ala Lys Pro Ser Arg Ser Arg Ala
                405                 410                 415

AAG GTG CTG ACA ACT GTG TGC TAA                                      1272
Lys Val Leu Thr Thr Val Cys
                420
```

(2) INFORMATION FOR SEQ ID NO:12:

(i) SEQUENCE CHARACTERISTICS:
(A) LENGTH: 423 amino acids
(B) TYPE: amino acid
(D) TOPOLOGY: linear

(ii) MOLECULE TYPE: protein

(xi) SEQUENCE DESCRIPTION: SEQ ID NO:12:

```
Met Asp Ser Gly Val Trp Ala Ala Cys Ile Phe Cys Leu Leu Ser Ser
1               5                   10                  15

Leu Pro Val Ala Leu Gly His Val His Pro Glu Cys Asp Phe Ile Thr
            20                  25                  30

Gln Leu Arg Glu Asp Glu Arg Thr Cys Leu Gln Ala Ala Asp Arg Met
        35                  40                  45

Ala Asn Ser Ser Ser Gly Cys Pro Arg Thr Trp Asp Gly Leu Leu Cys
        50                  55                  60

Trp Pro Thr Ala Gly Pro Gly Glu Trp Val Thr Leu Pro Cys Pro Ala
65                  70                  75                  80

Phe Phe Ser His Phe Ser Ser Glu Pro Gly Ala Leu Lys Arg Asp Cys
                85                  90                  95

Thr Thr Thr Gly Trp Ser Glu Pro Phe Pro Pro Tyr Pro Glu Ala Cys
                100                 105                 110
```

```
Pro Val Pro Leu Glu Leu Leu Thr Asp Glu Lys Ser Tyr Phe Ser Thr
        115                 120                 125

Val Arg Ile Val Tyr Thr Thr Gly His Ser Val Ser Ala Val Ala Leu
        130                 135                 140

Phe Val Ala Ile Ala Ile Leu Val Ala Leu Arg Arg Leu His Cys Pro
145                 150                 155                 160

Arg Asn Tyr Ile His Ser Gln Leu Phe Ala Thr Phe Ile Leu Lys Ala
            165                 170                 175

Gly Ala Val Phe Leu Lys Asp Ala Ala Leu Phe His Ser Glu Asn Thr
        180                 185                 190

Asp His Cys Ser Phe Ser Thr Val Leu Cys Lys Val Ser Val Ala Thr
        195                 200                 205

Ser His Phe Ala Thr Met Thr Asn Phe Ser Trp Leu Leu Ala Glu Ala
        210                 215                 220

Val Tyr Leu Thr Cys Leu Leu Ala Ser Thr Ser Pro Ser Thr Arg Arg
225                 230                 235                 240

Ala Phe Trp Trp Leu Val Leu Ala Gly Trp Gly Leu Pro Leu Leu Phe
            245                 250                 255

Thr Gly Thr Trp Val Gly Cys Lys Leu Ala Phe Glu Asp Val Ala Cys
            260                 265                 270

Trp Asp Leu Asp Asp Ser Ser Pro Tyr Trp Trp Ile Ile Lys Gly Pro
        275                 280                 285

Ile Val Leu Ser Val Gly Val Asn Phe Gly Leu Phe Leu Asn Ile Ile
        290                 295                 300

Arg Ile Leu Leu Arg Lys Leu Glu Pro Ala Gln Gly Ser Leu His Thr
305                 310                 315                 320

Gln Pro Gln Tyr Trp Arg Leu Ser Lys Ser Thr Leu Leu Leu Ile Pro
            325                 330                 335

Leu Phe Gly Ile His Tyr Val Ile Phe Asn Phe Leu Pro Asp Ser Ala
            340                 345                 350

Gly Leu Gly Ile Arg Leu Pro Leu Glu Leu Gly Leu Gly Ser Phe Gln
        355                 360                 365

Gly Phe Ile Val Ala Ile Leu Tyr Cys Phe Leu Asn Gln Glu Val Arg
        370                 375                 380

Thr Glu Ile Ser Arg Arg Trp His Gly His Asp Pro Glu Leu Leu Pro
385                 390                 395                 400
```

```
Ala Trp Arg Thr His Ala Lys Trp Ala Lys Pro Ser Arg Ser Arg Ala
              405                   410                   415

Lys Val Leu Thr Thr Val Cys
              420
```

**Claims**

1. The pGRF receptor.

2. A pGRF receptor of Claim 1 comprising the amino acid sequence: (Sequence ID#8)

```
His Val His Pro Glu Cys Asp Phe Ile Thr Gln Leu Arg Glu Asp Glu
Arg Thr Cys Leu Gln Ala Ala Asp Arg Met Ala Asn Ser Ser Ser Gly
Cys Pro Arg Thr Trp Asp Gly Leu Leu Cys Trp Pro Thr Ala Gly Pro
Gly Glu Trp Val Thr Leu Pro Cys Pro Ala Phe Phe Ser His Phe Ser
Ser Glu Pro Gly Ala Leu Lys Arg Asp Cys Thr Thr Thr Gly Trp Ser
Glu Pro Phe Pro Pro Tyr Pro Glu Ala Cys Pro Val Pro Leu Glu Leu
Leu Thr Asp Glu Lys Ser Tyr Phe Ser Thr Val Arg Ile Val Tyr Thr
Thr Gly His Ser Val Ser Ala Val Ala Leu Phe Val Ala Ile Ala Ile
Leu Val Ala Leu Arg Arg Leu His Cys Pro Arg Asn Tyr Ile His Ser
Gln Leu Phe Ala Thr Phe Ile Leu Lys Ala Gly Ala Val Phe Leu Lys
Asp Ala Ala Leu Phe His Ser Glu Asn Thr Asp His Cys Ser Phe Ser
Thr Val Leu Cys Lys Val Ser Val Ala Thr Ser His Phe Ala Thr Met
Thr Asn Phe Ser Trp Leu Leu Ala Glu Ala Val Tyr Leu Thr Cys Leu
Leu Ala Ser Thr Ser Pro Ser Thr Arg Arg Ala Phe Trp Trp Leu Val
Leu Ala Gly Trp Gly Leu Pro Leu Leu Phe Thr Gly Thr Trp Val Gly
Cys Lys Leu Ala Phe Glu Asp Val Ala Cys Trp Asp Leu Asp Asp Ser
Ser Pro Tyr Trp Trp Ile Ile Lys Gly Pro Ile Val Leu Ser Val Gly
Val Asn Phe Gly Leu Phe Leu Asn Ile Ile Arg Ile Leu Leu Arg Lys
Leu Glu Pro Ala Gln Gly Ser Leu His Thr Gln Pro Gln Tyr Trp Arg
Leu Ser Lys Ser Thr Leu Leu Leu Ile Pro Leu Phe Gly Ile His Tyr
Val Ile Phe Asn Phe Leu Pro Asp Ser Ala Gly Leu Gly Ile Arg Leu
Pro Leu Glu Leu Gly Leu Gly Ser Phe Gln Gly Phe Ile Val Ala Ile
Leu Tyr Cys Phe Leu Asn Gln Glu Val Arg Thr Glu Ile Ser Arg Arg
Trp His Gly His Asp Pro Glu Leu Leu Pro Ala Trp Arg Thr His Ala
Lys Trp Ala Lys Pro Ser Arg Ser Arg Ala Lys Val Ser Ala Cys Ser

Arg Ala Gly Ser Ser Arg Pro Arg Ala His Gly Asp Thr Tyr Pro Gly
Leu Glu Val Pro Gly Gln Trp Leu Cys Leu Phe Leu Thr,
```

and functional analogs thereof.

3.  A porcine GRF receptor of Claim 2 comprising the amino acid sequence (Sequence ID#12)

```
Met Asp Ser Gly Val Trp Ala Ala Cys Ile Phe Cys Leu Leu Ser Ser
Leu Pro Val Ala Leu Gly His Val His Pro Glu Cys Asp Phe Ile Thr
Gln Leu Arg Glu Asp Glu Arg Thr Cys Leu Gln Ala Ala Asp Arg Met
Ala Asn Ser Ser Ser Gly Cys Pro Arg Thr Trp Asp Gly Leu Leu Cys
Trp Pro Thr Ala Gly Pro Gly Glu Trp Val Thr Leu Pro Cys Pro Ala
Phe Phe Ser His Phe Ser Ser Glu Pro Gly Ala Leu Lys Arg Asp Cys
Thr Thr Thr Gly Trp Ser Glu Pro Phe Pro Pro Tyr Pro Glu Ala Cys
Pro Val Pro Leu Glu Leu Leu Thr Asp Glu Lys Ser Tyr Phe Ser Thr
Val Arg Ile Val Tyr Thr Thr Gly His Ser Val Ser Ala Val Ala Leu
Phe Val Ala Ile Ala Ile Leu Val Ala Leu Arg Arg Leu His Cys Pro
Arg Asn Tyr Ile His Ser Gln Leu Phe Ala Thr Phe Ile Leu Lys Ala
Gly Ala Val Phe Leu Lys Asp Ala Ala Leu Phe His Ser Glu Asn Thr
Asp His Cys Ser Phe Ser Thr Val Leu Cys Lys Val Ser Val Ala Thr
Ser His Phe Ala Thr Met Thr Asn Phe Ser Trp Leu Leu Ala Glu Ala
Val Tyr Leu Thr Cys Leu Leu Ala Ser Thr Ser Pro Ser Thr Arg Arg
Ala Phe Trp Trp Leu Val Leu Ala Gly Trp Gly Leu Pro Leu Leu Phe
Thr Gly Thr Trp Val Gly Cys Lys Leu Ala Phe Glu Asp Val Ala Cys
Trp Asp Leu Asp Asp Ser Ser Pro Tyr Trp Trp Ile Ile Lys Gly Pro
Ile Val Leu Ser Val Gly Val Asn Phe Gly Leu Phe Leu Asn Ile Ile
Arg Ile Leu Leu Arg Lys Leu Glu Pro Ala Gln Gly Ser Leu His Thr
Gln Pro Gln Tyr Trp Arg Leu Ser Lys Ser Thr Leu Leu Leu Ile Pro
Leu Phe Gly Ile His Tyr Val Ile Phe Asn Phe Leu Pro Asp Ser Ala
Gly Leu Gly Ile Arg Leu Pro Leu Glu Leu Gly Leu Gly Ser Phe Gln
Gly Phe Ile Val Ala Ile Leu Tyr Cys Phe Leu Asn Gln Glu Val Arg
Thr Glu Ile Ser Arg Arg Trp His Gly His Asp Pro Glu Leu Leu Pro
Ala Trp Arg Thr His Ala Lys Trp Ala Lys Pro Ser Arg Ser Arg Ala
Lys Val Leu Thr Thr Val Cys
```

4.  A DNA encoding the pGRF receptor of claim 1.

5.  The DNA of claim 4 wherein said DNA comprises the sequence: (Sequence ID#7)

```
CACGTGCACC CGGAATGTGA CTTCATCACC CAGCTGAGAG AAGACGAGCG

AACTTGTCTA CAAGCAGCAG ACCGGATGGC CAACTCCTCC TCGGGCTGTC

CTAGGACCTG GGATGGGCTG TTGTGCTGGC CGACGGCAGG CCCTGGGGAG

TGGGTGACCC TCCCCTGCCC GGCTTTCTTC TCTCACTTCA GCTCTGAGCC

AGGGGCCCTG AAGCGGGACT GCACCACCAC GGGCTGGTCT GAGCCCTTCC

CGCCATATCC CGAGGCTTGC CCTGTGCCCC TGGAGCTGCT GACTGATGAG

AAATCCTACT TCTCCACTGT GAGAATCGTC TACACCACGG GCCACAGCGT

CTCTGCCGTG GCCCTCTTCG TGGCCATCGC CATCCTGGTT GCTCTCAGGA

GGCTCCACTG CCCCAGGAAC TACATCCACA GCCAGCTGTT CGCCACCTTT

ATCCTCAAGG CGGGAGCTGT GTTCTTGAAA GACGCCGCCC TCTTTCACAG

CGAGAACACG GACCACTGCA GCTTCTCCAC GGTTCTGTGC AAGGTCTCTG

TGGCCACCTC CCATTTCGCC ACCATGACCA ACTTCAGCTG GCTGCTGGCA

GAAGCTGTCT ACCTGACCTG CCTCTTGGCC TCTACGTCAC CCAGCACGAG

GAGGGCCTTC TGGTGGCTGG TTCTCGCTGG CTGGGGGCTG CCCCTGCTCT

TCACTGGCAC GTGGGTGGGT TGCAAGTTGG CCTTTGAGGA TGTTGCGTGC

TGGGACCTGG ACGACAGCTC CCCCTACTGG TGGATCATCA AAGGGCCCAT

CGTCCTCTCC GTGGGGGTGA ACTTTGGGCT TTTTCTCAAT ATTATCCGCA

TCCTGCTGAG GAAACTGGAG CCAGCTCAGG GCAGCCTCCA CACCCAGCCT

CAGTACTGGC GTCTCTCCAA GTCAACCCTT CTCCTCATCC CGCTGTTTGG

AATTCACTAC GTCATCTTCA ACTTCCTGCC TGACAGTGCT GGCCTGGGCA

TCCGCCTCCC CCTGGAGCTG GGACTGGGCT CCTTCCAGGG CTTCATTGTT

GCCATCCTGT ACTGCTTCCT CAACCAAGAG GTGAGGACTG AGATCTCACG

GAGGTGGCAT GGCCATGACC CTGAACTTCT GCCAGCCTGG AGGACTCATG

CCAAATGGGC AAAGCCTTCC CGCTCAAGGG CGAAGGTCAG TGCGTGTTCA

CGTGCTGGGT CTTCCCGCCC CCGAGCCCAT GGCGACACAT ACCCGGGACT

GGAAGTGCCG GGTCAGTGGT TGTGCCTTTT CTTGACGTAG
```

6. A method for the recombinant production of a pGRF receptor, said method comprising the steps of:
   a) fabricating a DNA encoding the pGRF receptor,
   b) placing said DNA into an expression vector in a manner suitable for the expression of the pGRF receptor either alone or as a fusion protein,
   c) transforming an appropriate host cell with said expression vector,
   d) culturing said host cell under conditions to facilitate expression of said DNA, and
   e) recovering the recombinantly produced pGRF receptor.

7. A recombinant DNA vector comprising the DNA of Claim 3.

8. A recombinant host cell transformed with the vector of Claim 7.

9. A screening system for discovering agents which bind to the pGRF receptor said screening system comprising the steps of:
   a) producing a pGRF receptor according to the method of Claim 6,
   b) exposing said pGRF receptor to a potential inhibitor of the [pGRF-pGRF receptor] complex,
   c) introducing the pGRF,

d) removing non-specifically bound molecules, and

e) quantifying the concentration of bound potential inhibitor and/or pGRF.

10. A pGRF receptor bioactivity assay, said assay comprising the steps of:

a) transfecting a mammalian host cell with an expression vector comprising a DNA encoding a pGRF receptor and a signal peptide,

b) culturing said host cell under conditions such that the DNA encoding the pGRF receptor and signal peptide are expressed,

c) exposing said host cell so transfected to a test compound, and

d) quantifying the intracellular cAMP level in response to said test compound.

11. A transgenic animal comprising a DNA encoding the pGRF receptor.

FIG. I

FIG. 2

FIG. 3

FIG. 4

European Patent Office

**EUROPEAN SEARCH REPORT**

Application Number

EP 93 31 0446

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | NEUROPEPTIDES<br>vol. 25, no. 1 , July 1993<br>pages 1 - 10<br>HSIUNG HM;SMITH DP;ZHANG XY;BENNETT T;ROSTECK PR JR;LAI MH; 'Structure and functional expression of a complementary DNA for porcine growth hormone-releasing hormone receptor.'<br>* the whole document * | 1-11 | C12N15/12<br>C07K13/00<br>G01N33/68<br>A01K67/027 |
| A | WO-A-92 10565 (CORNELL RESEARCH FOUNDATION, INC.; US) 25 June 1992<br>* page 20, line 16 - page 20, line 25; claim 4 * | 1-10 | |
| A | MOLECULAR ENDOCRINOLOGY<br>vol. 6, no. 10 , October 1992<br>pages 1734 - 1744<br>MAYO, KE; 'Molecular cloning and expression of a pituitary-specific receptor for growth hormone-releasing hormone.' | | TECHNICAL FIELDS SEARCHED (Int.Cl.5)<br><br>C12N<br>G01N<br>A01K<br>C07K |

The present search report has been drawn up for all claims

| Place of search | Date of completion of the search | Examiner |
|---|---|---|
| THE HAGUE | 25 March 1994 | Nauche, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)